(19) Europäisches Patentamt
European Patent Office
Office européen des brevets

(11) EP 1 532 990 B1

(12) **EUROPEAN PATENT SPECIFICATION**

(45) Date of publication and mention of the grant of the patent:
**17.02.2010 Bulletin 2010/07**

(51) Int Cl.:
***A61L 9/01*** *(2006.01)* ***A61K 8/27*** *(2006.01)*
***C11D 3/50*** *(2006.01)*

(21) Application number: **04256993.9**

(22) Date of filing: **11.11.2004**

(54) **Deodorizing compositions containing zinc ricinoleate and at least one substituted monocyclic organic compound**

Zubereitungen mit desodorierender Wirkung, enthaltend das Zinksalz der Ricinolsäure und mindestens eine substituierte und monocyclische, organische Verbindung

Compositions désodorisantes contenant du ricinoléate de zinc et au moins un composé organique substitué et monocyclique

(84) Designated Contracting States:
**DE ES FR GB IT**

(30) Priority: **13.11.2003 US 706888**

(43) Date of publication of application:
**25.05.2005 Bulletin 2005/21**

(73) Proprietor: **INTERNATIONAL FLAVORS & FRAGRANCES INC.**
**New York**
**New York 10019 (US)**

(72) Inventors:
- **Parekh, Pradbodh P.**
  **Marlboro**
  **New Jersey 07746 (US)**
- **Nicoll, Stephen P.**
  **Ramsey**
  **New Jersey 07446 (US)**
- **Ramsammy, Vellidum**
  **Franklin**
  **New Jersey 07416 (US)**
- **Colt, Kristine K.**
  **Jackson**
  **New Jersey 08527 (US)**
- **Betz, Alison**
  **Middeltown**
  **New Jersey 07716 (US)**
- **Deshpande, Vikas M.**
  **Plainsboro**
  **New Jersey 08536 (US)**
- **Van Kippersluis, Wijnandra Hendrika**
  **Bussum (NL)**
- **Boden, Richard M.**
  **Ocean**
  **New Jersey 07712 (US)**

(74) Representative: **Mercer, Christopher Paul et al**
**Carpmaels & Ransford**
**43-45 Bloomsbury Square**
**London WC1A 2RA (GB)**

(56) References cited:
**EP-A- 1 167 507**
**WO-A-03/039505**
**US-A- 4 719 105**
**US-A1- 2003 199 402**
**US-B1- 6 242 509**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 532 990 B1

## Description

Synergistically-effective compositions comprising zinc ricinoleate or solutions thereof and

1-(4'-methylethyl)cyclohexylethan-1-yl propionate;

and uses of same and compositions containing same for preventing and/or suppressing malodors.

## BACKGROUND OF THE INVENTION

A wide variety of solid, liquid and gaseous functional materials including body deodorants, anti-perspirants, anti-perspirant/body deodorant devices, air fresheners which include air freshening devices, and solid and liquid air freshening and room freshening compositions, room deodorants, herbicides, antiviral compositions, fungicides, bactericides, parasiticides, insecticides, depilatory compositions, bleach compositions, hard surface-cleaning compositions, skin cleansing compositions, anti-microbial nail preparations including anti-fungal nail lacquers, hair setting compositions, hair conditioning compositions, trichological lotions, detergent compositions, soap compositions, sunscreen compositions, fabric stain-removal compositions, fabric softener compositions, fabric conditioning compositions, fabric anti-wrinkle compositions, skin softening compositions, skin texture enhancement compositions, such as line and wrinkle corrective compositions, skin lightening compositions, steam iron aroma compositions including stress relief compositions, candle compositions, plant growth regulating compositions, plant growth stimulating compositions, fertilizer compositions, insect attractant compositions, insect repelling compositions, drain cleaning compositions and molluskicide compositions have been developed that, although useful for their respective purposes, on use thereof emanate odours which are offensive to the human sense of smell.

In addition, a number of defined 3-dimensional spaces the use of which is required for various business and service operations and personal matters including indoor gymnasiums, indoor sporting event arenas, locker rooms, hair salons, nail salons, tanning salons, beauty salons, tattoo parlors, pig pens, chicken coops, cow barn enclosures, horse barn enclosures, indoor fresh fish markets, plant processing factory rooms, clothing dry cleaning rooms, garment laundry interiors, rooms containing in-use animal litter containers, abattoirs, cattle cars, zoo animal pens, morgues, autopsy rooms, lavatories, medical patient care rooms, hospital wards and dental patient care rooms have continuously prevailing odours which are offensive to the human sense of smell. Such odors which are offensive to the human sense of smell are caused by aliphatic halohydrins, aliphatic amines, aliphatic N-oxides, dialkylamines, cycloaliphatic amines, cycloaliphatic N-oxides, cyclo-olefinic amines, cyclo-olefinic N-oxides, cycloaromatic amines, cycloaromatic N-oxides, hydroxyalkylamines, imine compounds, amide compounds, amino acids, polypeptides, modified antimicrobial proteins, diureides, nitriles, aliphatic mercaptans, cycloaliphatic mercaptans, mercaptoalkanoic acids, mercaptoalkanoic acid esters, aliphatic monosulfides, disulfides, trisulfides, sulfur oxides, sulfones and sultones, cycloaliphatic monosulfides, disulfides, trisulfides, sulfur oxides, sulfones and sultones, cyclo-olefinic monosulfides, disulfides, trisulfides, sulfur oxides, sulfones and sultones, cycloaromatic monosulfides, disulfides, trisulfides, sulfur oxides, sulfones and sultones, alkali metal sulfites, bisulfites and metabisulfites, isothiocyanates, thiocyanates, dithiocyanates, isothiazolones, isothiazolinones, thiodiazinethiones, halosulfamates, aryl sulfonamides, lower aliphatic carboxylic acids, phenols, phosphines, aliphatic phosphites and phosphonates, cycloaliphatic phosphites and phosphonates, arsines, lower alcohols, lower ketones, hops, hops acids, aryl pyrazoles, oxazolines, isocyanurates, biguanides, extracts of krameria, hydantoins, pyrollidones, pyrollidone carboxylic acids, pyrollidone carboxylic acid esters, nitrophenols, N-substituted aspartic acids and pyrethroids. Compounds of these classes that have unpleasant odours are referred to herein as malodor compounds.

The aforementioned functional materials are known to include and/or have applied thereto or in a 3-dimensional space proximate thereto, fragrance materials that are intended to provide pleasant fragrances which mask the malodor.

In addition, the aforementioned defined 3-dimensional spaces are known to have introduced therein compositions such as fragrance materials that are intended to provide pleasant aromas which mask the malodor present therein and/or introduced thereto.

The masking effect is provided by one of three mechanisms. In the first mechanism, the masking composition components blend with the malodor compound or compounds in an effort to effect formation of a different and more desirable aroma. In the second mechanism the masking composition components are employed in a large quantity and concentration whereby the compound or composition responsible for the malodor is overwhelmed. In the third mechanism, one or more masking composition components overwhelm the compound or composition responsible for the malodor and one or more masking composition components chemically react with the malodor compound or composition thereby effecting formation of a more desirable aroma.

Each of the aforementioned mechanisms have serious disadvantages. Thus, in the case where a fragrance composition is used as a malodor maskant, it will not completely eliminate the perception of malodor and, accordingly, there is a tendency to use increasing quantities and concentrations of fragrance in an effort to eliminate the perception

of malodor. Furthermore, the masking effect is an additive effect and so the total odor level in the malodor-masked functional product or in the malodor-masked defined 3-dimensional space is increased by consumption of the fragrance. Even though the fragrance so used may be very pleasant at low concentration, the total odour level in the 3-dimensional space proximate the in-use functional product and the total odour level in the aforementioned defined 3-dimensional space at the relatively high concentrations required to achieve even moderate masking of the malodor will itself be offensive to the human sense of smell.

The prior art contains evidence of a large number of efforts to overcome the aforementioned disadvantages.

Thus, the use of zinc ricinoleate, also known as TEGO Sorb (Goldschmidt A.G. of Essen, Germany) for suppressing malodors is disclosed in U.S. Patent 6,528,047, U.S. Patent 6,592,813, U.S. Patent 4,968,496 and U.S. Patent Application 2003/007945 A1 published on January 9, 2003. Furthermore the use of one or more of the substituted cyclic organic compounds:

1-cyclohexylethan-1-yl butyrate;
1-cyclohexylethan-1-yl acetate;
1-cyclohexylethan-1-ol;
1-(4'-methylethyl)cyclohexylethan-1-yl propionate; and/or
2'-hydroxy-1-ethyl(2-phenoxy)acetate

sold under VEILEX (International Flavors & Fragrances Inc., New York, N.Y.) for suppressing malodors are disclosed in, for example, in U.S. Patent 6,432,891, U.S. Patent 6,592,813 and U.S. Patent 4,622,221.

Furthermore the use of zinc ricinoleate in functional substance such as fragrance containing polyamide sticks and packages dispensing same are disclosed in U.S. Patent Application No. 10/683,525 filed on October 10, 2003 but no mention is made therein of the use of polyamide sticks for malodor suppression or prevention.

However, nothing in the prior art discloses the synergistically-effective compositions of our invention; or such compositions being used to prevent and/or suppress malodors.

SUMMARY OF THE INVENTION

Our invention is directed to a synergistically-effective composition for preventing and/or suppressing malodors comprising:

(i) from about 10 to about 90 parts by weight of at least one substituted monocyclic organic compound-containing material, wherein the substituted monocyclic organic compound-containing material is:

1-(4'-methylethyl)cyclohexylethan-1-yl propionate having the structure:

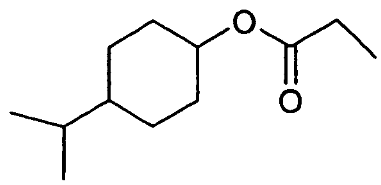

and

(ii) from about 90 to about 10 parts by weight of a zinc ricinoleate-containing composition selected from the group consisting of zinc ricinoleate and solutions of zinc ricinoleate containing > 30% by weight of zinc ricinoleate.

Preferably, the zinc ricinoleate-containing composition of our invention is a mixture of about 50% by weight of zinc ricinoleate and about 50% by weight of at least one 1-hydroxy-2-ethoxethyl ether of a $C_{12}$-$C_{14}$ fatty alcohol.

Our invention is also directed to a process for counteracting a malodor emanating from a solid or liquid malodorous source into a 3-dimensional space proximate said source (e.g., malodorous garbage contained in a polyethylene-fabricated garbage bag) comprising the step of introducing into the 3-dimensional space proximate said source a synergistically-effective malodor-counteracting quantity and concentration of the aforementioned zinc ricinoleate-substituted cyclic organic compound-containing composition of our invention as a single dose, as a continuous dose over a malodor-counteracting period of time, or as periodic doses over a malodor-counteracting period of time whereby the perceived total malodor intensity is substantially reduced or eliminated.

Our invention is also directed to a process for preventing a malodor from emanating from a solid or liquid malodorous source into a 3-dimensional space proximate said source comprising the step of admixing with said source a synergistically-effective malodor-counteracting quantity and concentration of the aforementioned zinc ricinoleate-substituted cyclic organic compound-containing composition of our invention.

More specifically our invention is directed to a process wherein the solid or liquid malodorous source evolving or capable of evolving the malodor is, for example, one of:

a herbicide; an antiviral composition; a fungicide; a bactericide; a parasiticide; an insecticide; a depilatory preparation; a bleach composition; a hard surface-cleaning preparation; a skin cleansing composition; an anti-microbial nail preparation; a hair setting composition; a hair conditioning composition; a trichological lotion; a skin lightening composition; a skin softening composition; a skin texture enhancement composition; a detergent composition; a soap composition; a sunscreen composition; a fabric stain removal composition; a fabric softening composition; a fabric conditioning composition; a fabric anti-wrinkle composition; a steam iron aroma composition; a candle composition; a plant growth regulating composition; a plant growth stimulating composition; a fertilizer composition; an insect attractant composition; an insect repelling composition; a drain cleaning composition; a molluskicide composition; an anti-perspirant composition; a body deodorant composition; a body deodorant/anti-perspirant device; a waste-disposal container; an air freshener device and an air freshener composition; and wherein the malodor is caused by, for example, a malodor-causing quantity and concentration of at least one compound selected from the group consisting of malodor compounds as described herein.

The terms system-compatible malodor-suppressing composition and system-compatible functional composition are herein intended to mean the malodor-suppressing compositions of our invention and other functional compositions, for example, fragrance compositions which, when made part of the support polymer-zinc ricinoleate/substituted monocyclic organic compound system do not compromise the transparency of the stick article by causing haze or cloudiness, due to, for example, phase separation or synerisis to occur as a result of the composition being admixed with the support polymer-zinc ricinoleate/substituted monocyclic organic compound system of our invention.

The term consistently-maintained malodor-suppressing composition and dimensional integrity is intended herein to mean that when the stick article of our invention is in use in applying a film to an inanimate solid laminar surface, the proportions of the constituents and the chemical properties of the malodor-suppressing composition of our invention and, optionally, other functional composition, such as a fragrance composition that is evolved into the environment on use of the stick article of our invention are substantially identical to the proportions and chemical properties of the constituents originally present in the stick article and originally admixed with the support polymer-zinc ricinoleate/substituted monocyclic organic compound system; and the geometric dimensions thereof with reference to the "x","y" and "z" axes on use thereof are substantially identical to the geometric dimensions originally existant in the stick article.

DETAILED DESCRIPTION OF THE INVENTION

The zinc ricinoleate component of the malodor-suppressing and/or preventing composition of our invention is preferably utilized in combination with a solvent which is at least one ethoxylated $C_{12}$-$C_{14}$ fatty alcohol, preferably one or more of a 1-hydroxy-2-ethoxyethyl ether of a $C_{12}$-$C_{14}$ fatty alcohol, for example, the 1-hydroxy-2-ethoxyethyl ether of stearyl alcohol in a concentration of greater than 30%, preferably at a concentration of about 50%.

In admixture with the zinc ricinoleate component of the malodor-suppressing and/or preventing composition of our invention as defined in claim 1, is optionally one or more additional substituted monocyclic organic chemicals.

A preferred substituted monocyclic organic compound composition useful in combination with the zinc ricinoleate component is a mixture of:

(A) 1-cyclohexylethan-1-yl butyrate;
(B) 1-cyclohexylethan-1-yl acetate; and
(C) 1-(4'-methylethyl)cyclohexylethan-1-yl propionate.

More preferably, the weight ratio of components of the immediately-aforementioned zinc riconoleate-substituted monocyclic organic compound mixture is one where the zinc ricinoleate-containing composition: 1-cyclohexylethan-1-yl butyrate: 1-cyclohexylethan-1-yl acetate: 1-(4'-methylethyl)cyclohexylethan-1-yl propionate is about 2:1:1:1.

Another preferred substituted monocyclic organic compound composition useful in combination with the zinc ricinoleate component is a mixture of:

(A) 1-cyclohexylethan-1-yl acetate; and
(B) 1-(4'-methylethyl)cyclohexylethan-1-yl propionate.

More preferably, the weight ratio of components of the immediately-aforementioned zinc riconoleate-substituted monocyclic organic compound mixture is one where the zinc ricinoleate-containing composition: 1-cyclohexylethan-1-yl acetate: 1-(4'-methylethyl)cyclohexylethan-1-yl propionate is about 3:1:1.

As stated above, the synergistically-effective compositions of our invention substantially eliminate the perception of malodors, and/or prevent the formation of such malodors while simultaneously refraining from reduction of the perception of pleasant fragrance aromas emanating from the same source or from the proximity of said source. The synergistically-effective zinc ricinoleate-substituted monocyclic organic compound compositions of our invention can be utilized with a vast number of functional products in order to prevent malodors evolved by such functional products during use thereof and/or to suppress malodors evolved by such functional products into the 3-dimensional space proximate the functional products while they are being used.

Examples of such functional products which are compositions, effective specific malodorous ingredients or classes of ingredients contained in and/or emanated from said functional products and exemplary defined air dimensional spaces where such functional products are used, together with U.S. Patent references setting forth specific examples of the utilities of such functional products are set forth in the following Table I:

TABLE I

| Functional Product | U. S. Patent Number | Ingredient or Ingredient Class | Defined Air 3-dimensional Space |
|---|---|---|---|
| hair fixative composition | 5,968,494 | 2,2-hydroxymethyl-substituted carboxylic acid | hair salons |
| hair fixative | 6,291,580 | polyurethanes and diisocyanates | hair salons |
| anti-microbial compositions | 6,294,186 | retinoids | health care facilities |
| Pesticides | 6,488,949 | thiacloprid | plant greenhouse |
| Laundry and cleaning compositions | 6,489,279 | xyloglucanase enzymes | laundries |
| Fungicides | 6,489,348 | substituted benzopyridyl ethers | hospital ward |
| Fungicides | 6,489,360 | phenyl benzyl ether imines, carbamates and dinitrophenols | plant greenhouse |
| Antibacterial liquid detergents | 6,492,313 | amine oxides and sodium paraffin sulfonates | kitchens |
| Cleaning wipes | 6,492,318 | amine oxides | kitchens |
| floor cleaning wipes | 6,492,499 | cocoamido-propyldimethyl amine oxide | kitchens |
| skin treatment compositions | 6,531,142 | sulfopolyester resins | health care facilities |
| Fabric care products | 6,531,444 | polyalkyleneimines | clothing dry cleaning rooms |
| Cleaning compositions | 6,465,404 | coconut diethanolamide | laundries |
| dish cleaning compositions | 6,465,406 | ethylenediamine-N,N-disuccinate | kitchens |
| Laundry detergents | 6,465,410 | N-vinylimidazole N-vinyl pyrrolidone copolymers | laundries |
| Pesticides | 6,468,555 | fluorophenyl oxazolines | plant greenhouses |
| Antimicrobial compositions | 6,471,974 | N-chlorosulfamates | health care facilities |
| Liquid cleaning compositions | 6,472,361 | sodium cumene sulfonate | kitchens |
| Biocide compositions | 6,475,505 | isothiazolinones | health care facilities |

(continued)

| Functional Product | U. S. Patent Number | Ingredient or Ingredient Class | Defined Air 3-dimensional Space |
|---|---|---|---|
| anti-viral compositions | 6,475,526 | t-butyl alkoxyphenols | health care facilities |
| Antimicrobial cosmetic formulations | 6,475,536 | extract of krameria | beauty salons |
| Antimicrobial compositions | 6,475,537 | hops acids | health care facilities |
| Cleaning compositions | 6,479,449 | triethanolamine | kitchens |
| Aerosol antimicrobial compositions | 6,482,392 | amine oxides | kitchens |
| Antimicrobial wipes | 6,482,423 | 2-pyrrolidone-5-carboxylic acid | kitchens |
| Parasiticidal compositions | 6,482,425 | imidazolyl pyridines | indoor farm facilities |
| Antimicrobial wipes | 6,488,943 | halogenated carbanilides | kitchens |
| Antibacterial compositions for skin care | 6,488,948 | behentrimonium methosulfate | beauty salons |
| nail care compositions | 6,503,488 | hydrogenated castor oil | beauty salons |
| Cosmetic and skin care sticks | 6,613,338 | benzimidazole sulfonic acids | beauty salons |

Preferably, the synergistically-effective zinc ricinoleate-substituted monocyclic organic compound-containing malodor preventing and/or suppressing compositions are employed in compatible solvents prior to the composition being incorporated into functional products. Such compatible solvents are, preferably, 1,2-propylene glycol, and di(1,2-dihydroxypropyl)ether, commonly known as dipropylene glycol or DPG. The range of dilution of the zinc ricinoleate-substituted monocyclic organic compound-containing malodor preventing and/or suppressing compositions in the compatible solvents is from about 2% to about 50% by weight of the zinc ricinoleate-containing composition of our invention, preferably from about 10% to about 30% and most preferably about 15% by weight of the zinc ricinoleate-containing composition of our invention.

When used in conjunction with malodorous solid or liquid functional products, e.g., soap and detergent compositions, the synergistically effective zinc ricinoleate-substituted monocyclic organic compound-containing malodor preventing and/or suppressing mixtures of our invention may be employed in a concentration of from about 75 ppm to about 300 ppm based on the weight of the solid or liquid composition preferably from about 100 ppm to about 250 ppm.

When used in conjunction with malodorous gaseous functional products, the synergistically effective zinc ricinoleate-substituted monocyclic organic compound-containing malodor preventing and/or suppressing mixtures of our invention may comprise from about 1.0 gram/liter to about 5.0 grams/liter, preferably from about 1.5 grams/liter to about 3.0 grams/liter of the air 3-dimensional space which contains the malodor to be counteracted.

As stated above, the functional products with which the zinc ricinoleate-substituted monocyclic organic compound compositions of our invention may be used also may optionally include fragrances, anti-microbial substances, animal repellents, insect attractants, and insect repellents, each of the components of which has a C $\log_{10}P$ in the range of from about 3.0 to about 8.0, without restriction on the molecular weight of each of said components wherein P is the n-octanol/water partition coefficient of the fragrance component. Examples of fragrance/anti-microbial compositions useful in the practice of our invention are set forth inU.S. Patent 6,495,512 and U.S. Patent 6,517,759.

The values of $C\log_{10}P$ of many functional product ingredients, for example, fragrance ingredients and/or anti-microbial ingredients, contained in personal treatment compositions and/or cosmetic compositions is discussed in U.S. Patent Application US 2003/0110682 A1 published on June 19, 2003, U.S. Patent 5,348,667, U.S. Patent 5,652,206 and U.S. Patent 6,528,013. Furthermore, values of $\log_{10}P$ have been reported; for example, the Pomona92 database, available from Daylight Chemical Information Systems, Inc., Daylight CIS, Irvine, California. However, the $\log_{10}P$ values are most conveniently calculated by the "CLOGP" program, also available from Daylight CIS. This program also lists experimental $\log_{10}P$ values when they are available in the Pomona92 database. The calculated $\log_{10}P$ ($C\log_{10}P$) is determined by the Hansch and Leo "fragment" approach based on the chemical structure of each perfume ingredient, and takes into account the numbers and types of atoms, the atom connectivity and the chemical bonding. The $C\log_{10}P$ values which are the most reliable and widely used estimates for this physicochemical property, are preferably used

instead of the experimental $log_{10}P$ values for the selection of functional ingredients, including perfume ingredients which are useful in the stick articles of our invention.

Specific examples of preferred fragrance components useful in the stick articles of our invention, and the molecular weights and $Clog_{10}P$ values of each of said components are set forth in Table II as follows:

Table II

| Fragance Component | $Clog_{10}P$ value | Molecular Weight |
|---|---|---|
| amyl salicylate | 4.601 | 208.26 |
| benzyl salicylate | 4.383 | 228.25 |
| β-caryophyllene | 6.333 | 204.36 |
| Cedrol | 4.530 | 222.37 |
| ethyl undecylenate | 4.888 | 212.34 |
| geranyl anthranilate | 4.216 | 273.38 |
| α-irone | 3.820 | 206.33 |
| phenyl ethyl benzoate | 4.058 | 226.28 |
| phenylethyl phenyl acetate | 3.767 | 240.31 |
| 1-phenyl hexanol-5 | 3.299 | 178.28 |
| α-santalol | 3.800 | 220.36 |
| amyl salicylate | 4.601 | 208.26 |
| benzyl salicylate | 4.383 | 228.25 |
| β-caryophyllene | 6.333 | 204.36 |
| Cedrol | 4.530 | 222.37 |
| cedryl acetate | 5.436 | 264.41 |
| cedryl formate | 5.070 | 238.37 |
| cyclohexyl salicylate | 5.265 | 220.29 |
| γ-dodecalactone | 4.359 | 198.31 |
| ethyl undecylenate | 4.888 | 212.34 |
| geranyl anthranilate | 4.216 | 273.38 |
| β-phenylethyl benzoate | 4.058 | 226.38 |
| β-phenylethyl phenyl acetate | 3.767 | 240.31 |
| 5-acetyl-1,1,2,3,3,6-hexamethyl indane | 5.977 | 258.41 |
| Cyclopentadecanolide | 6.246 | 240.39 |
| d-limonene | 4.232 | 136.24 |
| cis-p-t-butylcyclohexyl acetate | 4.019 | 198.31 |
| amyl cinnamic aldehyde | 4.324 | 202.30 |
| linalyl benzoate | 5.233 | 258.36 |

As stated above, the zinc ricinoleate-substituted monocyclic organic compound compositions of our invention may be employed in stick articles having an ETPA or ATPA polymeric structure.

Thus, the support polymer used in the stick article of our invention is, in the alternative or in combination (a) at least one ester-terminated polyamide or (b) at least one tertiary amide-terminated polyamide.

Preferable ester-terminated polyamides useful in the practice of our invention are those disclosed in U.S., Patent 5,783,657 and U.S. Patent 6,552,160 and include those ester-terminated polyamides prepared by reacting "x" equivalents of a dicarboxylic acid wherein at least 50% of those equivalents are from polymerized fatty acid, "y" equivalents

of ethylenediamine and "z" equivalents of an alcohol which is in the alternative, or in combination cetyl alcohol and/or stearyl alcohol wherein:

$$0.9 \le \frac{x}{y+z} \le 1.1 \text{ and } 0.1 \le \frac{z}{y+z} \le 0.7.$$

More preferably, the ester-terminated polyamide is one of a group having a weight-average molecular weight of about 6000 and a softening point in the range of from 88°C to 94°C prepared by reacting "x" equivalents of $C_{36}$ dicarboxylic acid, "y' equivalents of ethylenediamine and "z' equivalents of an alcohol which is, in the alternative or in combination cetyl alcohol and/or stearyl alcohol wherein

$$0.9 \le \frac{x}{y+z} \le 1.1 \text{ and } 0.1 \le \frac{z}{y+z} \le 0.7.$$

Most preferable are the mineral oil-free ester terminated polyamides, SYLVACLEAR 100LM, UNICLEAR 100 and UNICLEAR 100V, Arizona Chemical Company, Panama City, Florida.

Preferable tertiary amide-terminated polyamides useful in the practice of our invention are those disclosed in U.S.Patent 6,268,466 and U.S. Patent 6,469,131 and include those tertiary amide-terminated polyamides prepared by reacting "x" equivalents of dicarboxylic acid wherein at least 50% of those equivalents are from polymerized fatty acid, "y" equivalents of ethylenediamine and "z" equivalents of a monofunctional reactant having a secondary amine group as the only reactive functionality wherein

$$0.9 \le \frac{x}{y+z} \le 1.1 \text{ and } 0.1 \le \frac{z}{y+z} \le 0.7.$$

Most preferable are those tertiary amide-terminated polyamides disclosed in Example I of U.S. Patent 6,268,466.

BRIEF DESCRIPTION OF THE DRAWINGS

Figure 1 is a top view of a package useful for containing the malodor-suppressing stick article and for carrying out the process of applying the malodor-suppressing stick article to an inanimate solid laminar surface where the volatile components of the malodor-suppressing composition contained in the stick article is emitted via a controlled release mechanism.

Figure 2 is a cut-away side elevation view of the package of Figure 1.

Figure 3 is a perspective view of a second embodiment of the package showing the exposed stick article contained therein and ready for use.

Figure 4A shows a pair of comparative bar graphs showing malodor intensity, on the "Y" axis, on a scale of 0-9, for dry cloth initially having tobacco malodor treated with (a) unfragranced liquid detergent and (b) liquid detergent containing a mixture of 1-(4'-methylethyl)cyclohexylethan-1-yl propionate and zinc ricinoleate, 15% in dipropylene glycol.

Figure 4B shows a pair of comparative bar graphs showing malodor intensity, on the "Y" axis, on a scale of 0-9, for damp cloth initially having tobacco malodor treated with (a) unfragranced liquid detergent and (b) liquid detergent containing a mixture of 1-(4'-methylethyl)cyclohexylethan-1-yl propionate and zinc ricinoleate, 15% in dipropylene glycol.

Figure 5 shows a pair of comparative bar graphs showing panelist preference, on the "Y" axis, on a scale of 0-14, for damp cloth initially having tobacco malodor treated with (a) unfragranced liquid detergent and (b) liquid detergent containing a mixture of 1-(4'-methylethyl)cyclohexylethan-1-yl propionate and zinc ricinoleate, 15% in dipropylene glycol.

Figure 6 sets forth a schematic block flow diagram showing the steps from (a) creation of the zinc ricinoleate-substituted monocyclic organic compound-containing malodor-suppressing and/or preventing composition of our

invention to (b) the utilization by consumers of a liquid cosmetic base or detergent base containing the zinc ricinoleate-substituted monocyclic organic compound-containing malodor-suppressing and/or preventing composition of our invention.

Figure 7 sets forth a schematic block flow diagram showing the steps from (a) creation of the zinc ricinoleate-substituted monocyclic organic compound-containing malodor-suppressing and/or preventing composition of our invention to (b) the utilization by consumers of an aerosol product containing the zinc ricinoleate-substituted monocyclic organic compound-containing malodor-suppressing and/or preventing composition of our invention.

Figure 8 sets forth a schematic block flow diagram showing the steps from (a) creation of the zinc ricinoleate-substituted monocyclic organic compound-containing malodor-suppressing and/or preventing composition of our invention to (b) the application by consumers to a malodorous solid laminar surface of an ETPA or ATPA stick product containing the zinc ricinoleate-substituted monocyclic organic compound-containing malodor-suppressing and/or preventing composition of our invention.

DETAILED DESCRIPTION OF THE DRAWINGS

Referring to Figure 1 and Figure 2, the package 1 is adapted for use with the malodor-suppressing stick article comprising a removable cap 2 for closing the package to protect the product therein. The cap is removed to permit application of the malodor-suppressing product with optional fragrance composition contained in the ETPA and/or ATPA to an inanimate solid laminar surface, e.g., a garbage bag or porcelain sink surface. The package 1 further comprises a barrel 3 containing the malodor-suppressing stick article 4 of our invention. The wall of the barrel 3 closely surrounds the stick article 4. An applicator 5 having an upwardly facing applicator surface 6 is formed integrally with the barrel 3 at the top end thereof. The lower end or bottom 8 of the stick 4 is supported within the package on an oval-shaped, movable support member 9 for movement up or down within the package relative to the barrel 3. A central portion of the movable support member 9 is provided with a threaded coupling sleeve 10 for cooperation with an elevator screw 11. The lower end of the elevator screw is axially fixed but rotatable within an opening in the closed, bottom end of the barrel 3. The elevator screw 11 includes a tapered section 13 which can be snap fitted within the opening using resilient tabs 20, in the bottom of the barrel 3 to retain the elevator screw 11 in the position shown in Figure 2 while permitting the screw to be rotated by means of a knob 14 provided on the lower end of the screw. The bottom of the barrel 3 is dished inwardly to accommodate the knob 14 so that the package 1 can stand upright with the lower, outer, peripheral portion of the barrel, 3, resting on a flat supporting surface. Rotation of the knob 14 permits the user to raise or lower the movable support member 9 relative to the barrel 3 and thus raise and lower the malodor-suppressing stick article 4 of our invention relative to the barrel 3. The stick article 4 is shown in its lowered position in Figure 2 with the top of the stick flush with the applicator surface 6. The several components of the package 1, including the cap 2, barrel 3, applicator 5 and coupling sleeve 10 are preferably each formed of plastic, e.g., polypropylene or high density polyethylene including talc-filled polypropylene using techniques well-known to those skilled in the art. The outer surface portion of the applicator surface 6 is rounded, curved downward, for reducing drag during application of the polyamide-containing malodor-suppressing/optional fragrance (and/or additional functional ingredient, e.g., insect repellent) composition contained in the stick article to the inanimate solid laminar surface being treated. The outer surface portion ends in free end 15, which is below the applicator surface edge adjacent the barrel. The outer surface portion part that downwardly extends to free end 15 of the applicator is a cooperating surface upon which the lower skirt 16 of the cap 2 can be slidably fitted and removed with slight resistance. The cap 2 can have ribs 27 associated therewith to maintain the cap in a proper position relative to the barrel 3.

Referring to Figure 3, the malodor-suppressing stick article of our invention, 32, having application surface 33 is contained in package 30 which has a retaining wall 34 surrounding stick article 32. Holding wall 35, the purpose of which is to provide a convenient surface for an individual using package 30 in applying polyamide-containing malodor-suppressing/optional fragrance composition from stick article 33 to an inanimate solid laminar surface, e.g., a garbage bag or porcelain toilet bowl surface, surrounds retaining wall 34 in close proximity thereto and is juxtaposed thereto. As the stick article 32 is used during application to the inanimate solid laminar surface, it is moved by the user upward past retaining wall 34 using the rotatable manual positioning knob 31 which rotates about the vertical axis of package 30 as the stick article 32 moves up and down relative to retainer wall 34.

Figure 4A sets forth a pair of comparative bar graphs showing malodor intensity, on the "Y" axis, indicated by reference numeral 40, on a scale of 0-9, for dry cloth initially having tobacco malodor. The bar graphs are located along the "X" axis, indicated by reference numeral 41. The bar graph for the cloth treated with unfragranced liquid detergent is indicated by reference numeral 42. The bar graph for the cloth treated with liquid detergent containing a mixture of 1-(4'-methylethyl)cyclohexylethan-1-yl propionate and zinc ricinoleate, 15% in dipropylene glycol is indicated by reference numeral 43.

Figure 4B sets forth a pair of comparative bar graphs showing malodor intensity, on the "Y" axis, indicated by reference numeral 40, on a scale of 0-9, for damp cloth initially having tobacco malodor. The bar graphs are located

along the "X" axis, indicated by reference numeral 41. The bar graph for the cloth treated with unfragranced liquid detergent is indicated by reference numeral 44. The bar graph for the cloth treated with liquid detergent containing a mixture of 1-(4'-methylethyl)cyclohexylethan-1-yl propionate and zinc ricinoleate, 15% in dipropylene glycol is indicated by reference numeral 45.

Figure 5 sets forth a pair of comparative bar graphs showing panelist preference, on the "Y" axis, indicated by reference numeral 50, on a scale of 0-14, for damp cloth initially having tobacco malodor. The bar graphs are located along the "X" axis indicated by reference numeral 51. The bar graph for the cloth treated with unfragranced liquid detergent is indicated by reference numeral 52. The bar graph for the cloth treated with liquid detergent containing a mixture of 1-(4'-methylethyl)cyclohexylethan-1-yl propionate and zinc ricinoleate, 15% in dipropylene glycol is indicated by reference numeral 53.

Referring to the schematic block flow diagram of Figure 6, a solution, preferably a 50-50 solution of zinc ricinoleate and a 1-hydroxy-2-ethoxyethyl ether of a $C_{12}$-$C_{14}$ fatty alcohol in tank 61 is passed through line 62 past control valve 63 into vessel 70 equipped with agitator 71 where it is mixed, with agitation, with 1-(4'-methylethyl)cyclohexylethan-1-yl propionate and optionally one or more of the components:

1-cyclohexylethan-1-yl butyrate;
1-cyclohexylethan-1-yl acetate;
1-cyclohexylethan-1-ol;
and/or
2'-hydroxy-1'-ethyl(2-phenoxy)acetate

which is passed from tank 64 through line 65 past valve 66 into said vessel 70. Optionally, simultaneously, compatible diluent, e.g., propylene glycol or DPG, is passed from vessel 67 through line 68 past valve 69 into vessel 71. The resulting mixture is then passed through line 82 past valve 83 into vessel 84 equipped with agitator 85. Simultaneously, liquid cosmetic base or detergent base in vessel 77 is passed through line 78 past control valve 79 into vessel 84 where the detergent mixture is blended using agitator 85. Optionally, simultaneously, additional functional ingredient(s), e.g., compatible fragrance composition each of the components of which has a C $\log_{10}P$ of between 3.0 and 8.0 is passed from tank 72 (a) through line 73 past control valve 74 into vessel 70; (b) through line 75 past control valve 76 into vessel 77 for admixing with the cosmetic base or the detergent base and/or (c) through line 80 past valve 81 into vessel 84. The resulting mixture from vessel 84 is passed through conveyance 86 to location 87 whereat it is packaged. The packages are conveyed via conveyance 88 to marketing location 89 and marketed via marketing scheme 90 for ultimate consumer use, 91.

Referring to the schematic block flow diagram of Figure 7, a solution, preferably a 50-50 solution of zinc ricinoleate and a 1-hydroxy-2-ethoxyethyl ether of a $C_{12}$-$C_{14}$ fatty alcohol in tank 61 is passed into vessel 70 equipped with an agitator where it is mixed, with agitation, with 1-(4'-methylethyl)cyclohexylethan-1-yl propionate and optionally one or more of the components:

1-cyclohexylethan-1-yl butyrate;
1-cyclohexylethan-1-yl acetate;
1-cyclohexylethan-1-ol;
and/or
2'-hydroxy-1-ethyl(2-phenoxy)acetate

which is passed from tank 64 into said vessel 70. Optionally, simultaneously, compatible diluent, e.g., propylene glycol or DPG, is passed from vessel 67 into vessel 71. The resulting mixture is then passed through line 96 past valve 97 into aerosol packaging unit 95. Simultaneously, aerosol diluent, e.g., 1,1,1-trifluoroethane in pressure vessel 92 is passed through high pressure line 93 past control valve 94 into aerosol packaging unit 95 where the aerosol diluent mixture is inserted into pressurized aerosol cans at location 95. Optionally, simultaneously, additional functional ingredient(s), e.g., compatible fragrance composition each of the components of which has a C $\log_{10}P$ of between 3.0 and 8.0 is passed from tank 72 into vessel 70. The resulting filled aerosol cans are passed through conveyance 98 to location 99 whereat they are marketed for ultimate consumer use as malodor counteractant devices, 100.

Referring to the schematic block flow diagram of Figure 8, a solution, preferably a 50-50 solution of zinc ricinoleate and a 1-hydroxy-2-ethoxyethyl ether of a $C_{12}$-$C_{14}$ fatty alcohol in tank 61 is passed into vessel 70 equipped with an agitator where it is mixed, with agitation, with 1-(4'-methylethyl)cyclohexylethan-1-yl propionate and optionally one or more of the components:

1-cyclohexylethan-1-yl butyrate;
1-cyclohexylethan-1-yl acetate;
1-cyclohexylethan-1-ol;
and/or
2'-hydroxy-1'-ethyl(2-phenoxy)acetate

which is passed from tank 64 into said vessel 70. The resulting mixture is then passed through line 106 past valve 107 into vessel 110 equipped with an agitator. Simultaneously, ester-terminated polyamide (ETPA) and/or amide-terminated

polyamide (ATPA) in vessel 101 is passed through line 104 past control valve 105 into vessel 110 where the ETPA and/or ATPA-VEILEX-malodor mixture is blended. Optionally, simultaneously, additional functional ingredient(s), e.g., compatible fragrance composition each of the components of which has a C $log_{10}P$ of between 3.0 and 8.0 is passed from tank 72 (a) into vessel 70; (b) through line 102 past control valve 103 into vessel 101 for admixing with the ETPA and/or the ATPA and/or (c) through line 108 past valve 109 into vessel 110. The resulting mixture from vessel 110 is passed through conveyance 111 past control valve 112 to stick molding operation 113 whereat the ATPA and/or ETPA-malodor blend is formed into malodor prevention and/or suppression sticks. The resulting sticks are passed via conveyance 114 to packaging operation 115. The resulting packages created at 115, for example, those shown in Figures 1, 2 and 3 are conveyed via conveyance 116 to marketing location 117 and marketed via marketing scheme 118 for ultimate consumer use, 119, e.g., application of the stick to a malodorous solid laminar surface such as the outer surface of a polyethylene-fabricated garbage bag containing malodorous garbage or other solid malodorous waste, e.g., kitty litter.

The following examples are not meant to define or otherwise limit the scope of the invention. Rather the scope of the invention is to be ascertained according to the claims that follow the examples. Unless noted to the contrary, all percentages are given on a weight percent on a dry basis.

EXAMPLE A

The following fragrance composition was prepared:

| **Fragrance Component** | $Clog_{10}P$ value | Molecular Weight | Parts by Weight |
|---|---|---|---|
| ethyl undecylenate | 4.888 | 212.34 | 3.0 |
| geranyl anthranilate | 4.216 | 273.38 | 7.5 |
| α-irone | 3.820 | 206.33 | 6.3 |
| phenyl ethyl benzoate | 4.058 | 226.28 | 3.2 |
| d-limonene | 4.232 | 136.24 | 3.2 |
| cis-p-t-butylcyclohexyl acetate | 4.019 | 198.31 | 5.8 |
| amyl cinnamic aldehyde | 4.324 | 202.30 | 7.3 |
| hexyl cinnamic aldehyde | 5.473 | 216.33 | 12.6 |
| hexyl salicylate | 5.260 | 222.29 | 12.6 |

EXAMPLE B

The following fragrance was prepared:

| **Fragrance Component** | $Clog_{10}P$ value | Molecula r Weight | Parts by Weight |
|---|---|---|---|
| ethyl undecylenate | 4.888 | 212.34 | 10.5 |
| geranyl anthranilate | 4.216 | 273.38 | 35.4 |
| α-irone | 3.820 | 206.33 | 5.3 |
| phenyl ethyl benzoate | 4.058 | 226.28 | 5.3 |
| phenylethyl phenyl acetate | 3.767 | 240.31 | 5.3 |
| 5-acetyl- 1,1,2,3,3,6-hexamethyl indane | 5.977 | 258.41 | 2.5 |
| cyclopentadecanolide | 6.246 | 240.39 | 7.5 |
| d-limonene | 4.232 | 136.24 | 25.0 |
| cis-p-t-butylcyclohexyl acetate | 4.019 | 198.31 | 4.0 |
| amyl cinnamic aldehyde | 4.324 | 202.30 | 4.0 |

In each of the following Examples I-IV, fragrance formulation and a mixture in the ratio of 2 parts by weight of

a 50% solution of zinc ricinoleate in a 1-hydroxy-2-ethoxyethyl ether of a $C_{12}$-$C_{14}$ fatty alcohol, referred to herein as TEGO Sorb Conc. 50:1 part by weight of one or more VEILEX compounds, such as 1-(4'-methylethyl)-cyclohexylethan-1-yl propionate was premixed and added, with stirring to UNICLEAR-100-LM ester-terminated polyamide at a temperature of 40°C. The resulting mixture was then placed into molds designed to produce stick articles which fit into packages as illustrated in Figure 3. The resulting packaged stick articles were then employed in coating malodor-suppressing and fragrance-emitting films on garbage bags initially emitting intense garbage malodors. The resulting scaled value "V' on a scale of 0-10 (where $V = \frac{V_M^{-1} + V_F}{2}$ wherein $V_M$ is a scaled value representing malodor intensity and $V_F$ is a scaled value representing fragrance intensity and pleasantness) for each of the coatings is set forth in each of the examples; and is in the range of 7.5≤ V ≤ 10. Those with skill in the art will appreciate that a higher score is superior to a lower score, for example a score of 10 is superior to a score of 7.5.

EXAMPLE I

The following mixtures were prepared and molded into stick articles as set forth above:

| Ingredients | Parts by Weight | | | |
|---|---|---|---|---|
| | Example IA | Example IB | Example IC | Example ID |
| Fragrance of Example A, supra | 30.0 | 0 | 30.0 | 30.0 |
| Fragrance of Example B, supra | 0 | 30.0 | 0 | 0 |
| UNICLEAR 100-LM | 67.5 | 67.5 | 68.25 | 68.0 |
| 1-(4'-methylethyl)cyclohexylethan-1-yl propionate | 2.0 | 2.0 | 1.20 | 1.50 |
| TEGO Sorb Conc.50 | 4.0 | 4.0 | 2.40 | 3.00 |

The "V" value of the resulting coatings for each of Examples IA, IB, IC and ID is 9.75. This example demonstrates the improvement of the combination of the TEGO sorb and malodor blocking materials in view of the malodor blocking materials alone.

EXAMPLE II

The following mixtures was prepared and molded into stick articles as set forth above:

| Ingredients | Parts by Weight | |
|---|---|---|
| | Example IIA | Example IIB |
| Fragrance of Example A, supra | 30.0 | 30.0 |
| UNICLEAR 100-LM | 69.50 | 69.75 |
| 50:50 mixture of 1-cyclohexylethan-1-yl butyrate and 1-cyclohexylethan-1-yl acetate | 0.50 | 1.50 |
| TEGO Sorb Conc.50 | 1.0 | 3.00 |

The "V" value of the resulting coating for Example IIA is 7.75. The "V" value of the resulting coating for Example IIB is 8.10. This example demonstrates the superiority and the synergism of the combination of the TEGO Sorb when used with a 50:50 mixture of 1-cyclohexylethan-1-yl butyrate and 1-cyclohexylethan-1-yl acetate, as compared to using the 50:50 mixture alone or using the TEGO Sorb material alone.

EXAMPLE III

The following mixtures were prepared and molded into stick articles as set forth above:

| Ingredients | Parts by Weight | |
|---|---|---|
| | Example IIIA | Example IIIB |
| Fragrance of example B, supra | 30.0 | 30.0 |
| UNICLEAR 100-LM | 69.50 | 69.75 |
| 30:30:40 mixture of 1-cyclohexylethan-1-yl butyrate, 1-cyclohexylethan-1-yl acetate and 2'-hydroxy-1'-ethyl(2-phenoxy)acetate | 3.00 | 4.00 |
| TEGO Sorb Conc.50 | 6.00 | 8.00 |

The "V" value of the resulting coating for Example IIIA is 8.65. The "V" value of the resulting coating for Example IIIB is 8.35.

The same examples are carried out (a) in the absence of the TEGO Sorb but in the presence of the 30:30:40 mixture of 1-cyclohexylethan-l-yl butyrate, 1-cyclohexylethan-1-yl acetate and 2'-hydroxy-1'-ethyl(2-phenoxy)acetate and (b) in the absence of any known malodor blocking compounds but in the presence of the TEGO Sorb. In each example that is carried out in the absence of TEGO Sorb and in the presence of the 30:30:40 mixture of 1-cyclohexylethan-1-yl butyrate, 1-cyclohexylethan-1-yl acetate and 2'-hydroxy-1'-ethyl(2-phenoxy)acetate the "V" values are 3.6 for Example IIIA without the TEGO Sorb and 3.8 for example IIIB without the TEGO Sorb. In each example that is carried out in the absence of any known malodor blocking compound but in the presence of TEGO Sorb, the "V" values are 4.65 without any known malodor blocking compound for Example IIIA and 4.55 without any known malodor blocking compound for Example IIIB. This example demonstrates the superiority and synergism of the combination of TEGO Sorb when used with a 50:50 mixture of 1-cyclohexylethan-1-yl butyrate and 1-cyclohexylethan-1-yl acetate, as opposed to using a 50:50 mixture of 1-cyclohexylethan-1-yl butyrate and 1-cyclohexylethan-1-yl acetate alone, or using TEGO Sorb, alone.

## EXAMPLE IV

The following mixtures were prepared and molded into stick articles as set forth above:

| Ingredients | Parts by Weight | | |
|---|---|---|---|
| | Example IVA | Example IVB | Example IVC |
| Fragrance of example A, supra | 30.0 | 30.0 | 30.0 |
| UNICLEAR 100-LM | 69.00 | 65.00 | 60.00 |
| 10:10:10:10 mixture of 1-cyclohexylethan-1-yl butyrate, 1-cyclohexylethan-1-yl acetate, 1-(4'-methylethyl)cyclohexylethan-1-yl propionate and 2'-hydroxy-1'-ethyl(2-phenoxy)acetate | 5.00 | 3.00 | 1.50 |
| TEGO Sorb Conc 50 | 10.00 | 6.00 | 3.00 |

The "V" value of the resulting coating for Example IVA is 8.75. The "V" value of the resulting coating for Example IVB is 8.90. The "V" value of the resulting coating for Example IVC is 9.10.

The same examples are carried out (a) in the absence of the TEGO Sorb but in the presence of the 10:10:10:10 mixture of 1-cyclohexylethan-1-yl butyrate, 1-cyclohexylethan-1-yl acetate, 1-(4'-methylethyl)cyclohexylethan-1-yl propionate and 2'-hydroxy-1'-ethyl(2-phenoxy)acetate and (b) in the absence of any malodour blocking compounds but in the presence of the TEGO Sorb. In each example that is carried out in the absence of TEGO Sorb and the presence of the 10:10:10:10 mixture of 1-cyclohexylethan-1-yl butyrate, 1-cyclohexylethan-1-yl acetate, 1-(4'-methylethyl)cyclohexylethan-1-yl propionate and 2'-hydroxy-1'-ethyl(2-phenoxy)acetate the "V" values are 3.9 for Example IVA without the TEGO Sorb, 3.8 for Example IVB without the TEGO Sorb and 3.65 for Example IVC without the TEGO Sorb. In each example that is carried out in the absence of any malodor blocking compound but in the presence of TEGO Sorb, the "V" values are 4.95 without any malodor blocking compound for Example IVA, 4.85 without any malodor blocking compound for Example IVB and 4.70 without any malodor blocking compound for Example IVC. This example demonstrates the superiority and synergism of the combination of TEGO Sorb when used with a 10:10:10:10 mixture of 1-cyclohexylethan-1-yl butyrate, 1-cyclohexylethan-1-yl acetate, 1-(4'-methylethyl)cyclohexylethan-1-yl propionate and 2'-hydroxy-1'-ethyl(2-phenoxy)acetate, as opposed to using a 10:10:10:10 mixture of 1-cyclohexylethan-1-yl butyrate, 1-cyclohexylethan-1-yl acetate, 1-(4'-methylethyl)cyclohexylethan-1-yl propionate and 2'-hydroxy-1'-ethyl(2-phenoxy)acetate alone, or using TEGO Sorb, alone. This example also demonstrates that when using the combination of TEGO Sorb

with a 10:10:10:10 mixture of 1-cyclohexylethan-1-yl butyrate, 1-cyclohexylethan-1-yl acetate, 1-(4'-methylethyl)cyclohexylethan-1-yl propionate and 2'-hydroxy-1'-ethyl(2-phenoxy)acetate, a lower concentration of the combination in a molded stick article, such as about 4.75% was found to be more effective than a higher concentration of the combination, such as about 13%.

EXAMPLE V

Two 1 foot square damp cotton cloth swatches (Identified as Swatch A and Swatch B) and two 1 foot square dry cotton cloth swatches (Identified as Swatch C and Swatch D) were each exposed to cigar smoke evolved from 4 King Edward VII fresh cigars in four separate 3 $ft^3$ air-tight enclosed chambers for a period of 12 hours thereby imparting an intense tobacco malodor to each swatch. The four swatches were then removed from the chambers and each swatch was separately washed for a wash time of 6 minutes and a rinse time of 2 minutes as follows:
(a) Swatch A and Swatch C:
A liquid detergent composition was prepared as described in Example 4 on Page 6 of U.S. Patent Application US 2003/0114323 A1 published on June 19, 2003, containing 99.775% detergent base (HLB Value of 13.2) and 0.225% of the following mixture:

| Ingredients | Parts by Weight |
|---|---|
| TEGO Sorb. Conc.50 | 10 |
| 1-(4'-methylethyl)cyclohexylethan-1-yl propionate | 5 |
| Dipropylene Glycol | 85 |

(b) Swatch B and Swatch D:
A liquid detergent composition as described in Example 4 on Page 6 of U.S. Patent Application US 2003/0114323 A1 published on June 19, 2003, containing 100% detergent base (HLB Value of 13.2).
Each of the swatches were then measured by a 10 member expert panel for tobacco malodor intensity, scaled on a scale of 0-9, and the results are summarized in Figures 4A and 4B, described above and set forth on the following table. In addition, the damp cloth swatches, A and B were measured for preference by a 10 member experienced panel, scaled on a scale of 0-14:

| Cloth Swatch Identification | Malodor Intensity Scaled Value (Figures 4A and 4B) | Panelist Preference Scaled Value (Figure 5) |
|---|---|---|
| A (Damp Cloth-Fig.4B, Reference Numeral 45 and Fig.5, Reference Numeral 53) | 2.3 | 12 |
| B (Damp Cloth-Fig.4B, Reference Numeral 44 and Fig. 5, reference numeral 52) | 8.0 | 5.2 |
| C (Dry Cloth-Fig.4A, Reference Numeral 43) | 1.3 | ---- |
| D (Dry Cloth-Fig.4A, Reference Numeral 42) | 7.7 | ---- |

Based upon the scoring criteria set forth above, a lower score is desirable for the malodor intensity and a higher score is more desirable for panelist preference. The test results reported above are consistent in their result. With the combination of TEGO Sorb and malodor blocking compounds, Swatches A and C, the resulting malodor intensity is low and the panelist preference is high. When the TEGO Sorb material is not provided, Swatches B and D, the resulting malodor intensity is high and the panelist preference is low.

## Claims

1. A composition comprising:

(i) from 10 to 90 parts by weight of a substituted monocyclic organic compound-containing material, wherein the substituted monocyclic organic compound-containing material is 1-(4'-methylethyl)cyclohexylethan-1-yl propionate ; and
(ii) from 90 to 10 parts by weight of a zinc ricinoleate-containing composition selected from the group consisting of zinc ricinoleate and a zinc ricinoleate solution containing greater than 30% by weight of zinc ricinoleate.

**2.** The composition of claim 1 further comprising an additional substituted monocyclic organic compound-containing material selected from the group consisting of 1-cyclohexylethan-1-yl butyrate, 1-cyclohexylethan-1-yl acetate and 1-cyclohexylethan-1-ol.

**3.** The composition of claim 1 or claim 2 wherein the zinc ricinoleate-containing composition of part (ii) is a zinc ricinoleate solution containing about 50% by weight of zinc ricinoleate and about 50% by weight of at least one 1-hydroxy-2-ethoxethyl ether of a $C_{12}$ - $C_{14}$ alcohol.

**4.** The composition of claim 1, comprising a mixture of 1-cyclohexylethan-1-yl butyrate and 1-cyclohexylethan-1-yl acetate.

**5.** The composition of claim 4 wherein the zinc ricinoleate-containing composition defined in part (ii) of claim 1 is the zinc ricinoleate solution containing about 50% by weight of zinc ricinoleate and about 50% by weight of at least one 1-hydroxy-2-ethoxethyl ether of a $C_{12}$-$C_{14}$ alcohol and wherein the weight ratio of zinc ricinoleate-containing composition to 1-cyclohexylethan-1-yl butyrate to 1-cyclohexylethan-1-yl acetate to 1-(4'-methylethyl)cyclohexylethan-1-yl propionate is about 2:1:1:1.

**6.** The composition of claim 1, comprising 1-cyclohexylethan-1-yl acetate.

**7.** The composition of claim 6 wherein the zinc ricinoleate-containing composition defined in part (ii) of claim 1 is the zinc ricinoleate solution containing about 50% by weight of zinc ricinoleate and about 50% by weight of at least one 1-hydroxy-2-ethoxethyl ether of a $C_{12}$-$C_{14}$ alcohol and wherein the weight ratio of zinc ricinoleate-containing composition to 1-cyclohexylethan-1-yl acetate to 1-(4'-methylethyl)cyclohexylethan-1-yl propionate is about 3:1:1.

**8.** A process for substantially reducing, eliminating, or counteracting a malodor emanating from a solid or liquid malodorous source
comprising the step of providing a malodor-counteracting quantity and concentration of the composition of any one of claims 1 to 7 as a single dose, a continuous dose over a malodor-counteracting period of time, or a periodic dose.

**9.** A process for preventing a malodor from emanating from a solid malodorous source having a defined laminar surface comprising the step of coating onto said defined laminar surface a malodor-counteracting quantity and concentration of the composition of any one of claims 1 to 7.

**10.** A process for preventing a malodor from emanating from a solid or liquid malodorous source comprising the step of admixing with said source a malodor-counteracting quantity and concentration of the composition of any one of claims 1 to 7.

**11.** The process of any one of claims 8 to 10, wherein the malodorous source is: a herbicide, an antiviral composition, a fungicide, a bactericide, a parasiticide, an insecticide, a depilatory preparation, a bleach composition, a hard surface-cleaning preparation, a skin cleansing composition, an anti-microbial nail preparation; a hair setting composition, a hair conditioning composition, a trichological lotion; a skin softening composition, a skin texture enhancement composition, a skin lightening composition, a detergent composition, a soap composition, a sunscreen composition, a fabric stain removal composition, a fabric softener composition, a fabric conditioning composition, a fabric anti-wrinkle composition, a steam iron aroma composition, a candle composition, a plant growth regulating composition, a plant growth stimulating composition, a fertilizer composition, an insect attractant composition, an insect repelling composition, a drain cleaning composition, a molluskicide composition; an anti-perspirant composition; a body deodorant composition, a body deodorant/anti-perspirant device, a waste-disposal container, an air freshener device or an air freshener composition.

**12.** The process of claim 11, wherein the malodorous source is an aliphatic halohydrin, aliphatic amine, aliphatic N-oxide, dialkylamine, cycloaliphatic amine, cycloaliphatic N-oxide, cyclo-olefinic amine, cyclo-olefinic N-oxide, cycloaromatic amine, cycloaromatic N-oxide, hydroxyalkylamine, imine compound, amide compound, amino acid,

polypeptide, modified antimicrobial protein, diureide, nitrile, aliphatic mercaptan, cycloaliphatic mercaptan, mercaptoalkanoic acid, mercaptoalkanoic acid ester, aliphatic monosulfide, disulfide, trisulfide, sulfur oxide, sulfone or sultone, cycloaliphatic monosulfide, disulfide, trisulfide, sulfur oxide, sulfone or sultone, cyclo-olefinic monosulfide, disulfide, trisulfide, sulfur oxide, sulfone or sultone, cycloaromatic monosulfide, disulfide, trisulfide, sulfur oxide, sulfone or sultone, isothiocyanate, thiocyanate, dithiocyanate, isothiazolone, isothiazolinone, thiodiazinethione, halosulfamate, aryl sulfonamide, lower aliphatic carboxylic acid, phenol, phosphine, aliphatic phosphite or phosphonate, cycloaliphatic phosphite or phosphonate, arsine, lower alcohol, lower ketone, hops, hop acid, aryl pyrazole, oxazoline, isocyanurate, biguanide, extract of krameria, hydantoin, pyrollidone, pyrollidone carboxylic acid, pyrollidone carboxylic acid ester, nitrophenol, N-substituted aspartic acid or pyrethroid.

**13.** A method for counteracting a malodor emanating from a solid or liquid fragrance-containing soap or detergent comprising the step of introducing into the soap or detergent a malodor-counteracting quantity and concentration of the composition of any one of claims 1 to 7.

**14.** A single liquid phase composition consisting essentially of from 10 to 30% by weight of the composition of any one of claims 1 to 7 and a solvent of glycol or dipropylene glycol.

**15.** A soap or detergent comprising the composition of any one of claims 1 to 7 wherein the concentration of the composition is from 75 ppm to 300 ppm.

**Patentansprüche**

**1.** Zusammensetzung, umfassend:

(i) von 10 bis 90 Gewichtsteile eines Materials, das eine substituierte monocyclische organische Verbindung enthält, wobei das Material, das eine substituierte monocyclische organische Verbindung enthält, 1-(4'-Methylethyl)cyclohexylethan-1-ylpropionat ist; und
(ii) von 90 bis 10 Gewichtsteile einer Zinkricinoleat enthaltenden Zusammensetzung, ausgewählt aus der Gruppe, bestehend aus Zinkricinoleat und einer Zinkricinoleat-Lösung, die mehr als 30 Gewichtsprozent Zinkricinoleat enthält.

**2.** Zusammensetzung nach Anspruch 1, ferner umfassend ein zusätzliches Material, das eine substituierte monocyclische organische Verbindung enthält, ausgewählt aus der Gruppe, bestehend aus 1-Cyclohexylethan-1-ylbutyrat, 1-Cyclohexylethan-1-ylacetat und 1-Cyclohexylethan-1-ol.

**3.** Zusammensetzung nach Anspruch 1 oder 2, wobei die Zinkricinoleat enthaltende Zusammensetzung aus Teil (ii) eine Zinkricinoleat-Lösung ist, die etwa 50 Gewichtsprozent Zinkricinoleat und etwa 50 Gewichtsprozent mindestens eines 1-Hydroxy-2-ethoxyethylethers eines $C_{12}$-$C_{14}$-Alkohols enthält.

**4.** Zusammensetzung nach Anspruch 1, umfassend ein Gemisch aus 1-Cyclohexylethan-1-ylbutyrat und 1-Cyclohexylethan-1-ylacetat.

**5.** Zusammensetzung nach Anspruch 4, wobei die Zinkricinoleat enthaltende Zusammensetzung, definiert in Teil (ii) von Anspruch 1, die Zinkricinoleat-Lösung ist, die etwa 50 Gewichtsprozent Zinkricinoleat und etwa 50 Gewichtsprozent mindestens eines 1-Hydroxy-2-ethoxyethylethers eines $C_{12}$-$C_{14}$-Alkohols enthält, wobei das Gewichtsverhältnis von Zinkricinoleat enthaltender Zusammensetzung zu 1-Cyclohexylethan-1-ylbutyrat zu 1-Cyclohexylethan-1-ylacetat zu 1-(4'Methylethyl)cyclohexylethan-1-ylpropionat etwa 2:1:1:1 beträgt.

**6.** Zusammensetzung nach Anspruch 1, umfassend 1-Cyclohexylethan-1-ylacetat.

**7.** Zusammensetzung nach Anspruch 6, wobei die Zinkricinoleat enthaltende Zusammensetzung, definiert in Teil (ii) von Anspruch 1, die Zinkricinoleat-Lösung ist, die etwa 50 Gewichtsprozent Zinkricinoleat und etwa 50 Gewichtsprozent mindestens eines 1-Hydroxy-2-ethoxyethylethers eines $C_{12}$-$C_{14}$-Alkohols enthält, wobei das Gewichtsverhältnis von Zinkricinoleat enthaltender Zusammensetzung zu 1-Cyclohexylethan-1-ylacetat zu 1-(4'Methylethyl)-cyclohexylethan-1-ylpropionat etwa 3:1:1 beträgt.

**8.** Verfahren zur wesentlichen Reduzierung, Eliminierung oder Entgegenwirken eines Gestankes, hervorgerufen von

einer festen oder flüssigen übelriechenden Quelle, umfassend
den Schritt des Bereitstellens einer Menge und Konzentration der Zusammensetzung nach einem der Ansprüche 1 bis 7, die dem Gestank entgegenwirkt, in einer einzigen Dosis, in einer kontinuierlichen Abgabe über einen Zeitraum, solange dem Gestank entgegengewirkt wird, oder in einer regelmäßigen Dosierung.

**9.** Verfahren zur Verhinderung, dass ein Gestank aus einer festen übelriechenden Quelle, die eine definierte laminare Oberfläche aufweist, austreten kann, umfassend den Schritt des Beschichtens der definierten laminaren Oberfläche mit einer Menge und Konzentration der Zusammensetzung nach einem der Ansprüche 1 bis 7, die dem Gestank entgegenwirkt.

**10.** Verfahren zur Verhinderung, dass ein Gestank aus einer festen oder flüssigen übelriechenden Quelle austreten kann, umfassend den Schritt des Beimischens einer Menge und Konzentration der Zusammensetzung nach einem der Ansprüche 1 bis 7, die dem Gestank entgegenwirkt, zu der Quelle.

**11.** Verfahren nach einem der Ansprüche 8 bis 10, wobei die übelriechende Quelle: ein Herbizid, eine antivirale Zusammensetzung, ein Fungizid, ein Bakterizid, ein Parasitizid, ein Insektizid, ein Haarentfernungspräparat, eine Bleichmittelzusammensetzung, eine Hartoberflächen-reinigende Formulierung, eine Hautreinigungsmittelzusammensetzung, ein antimikrobielles Nagelpräparat, eine Haarfixierungsmittelzusammensetzung, eine Haar-Konditionierungs-Mittelzusammensetzung, eine trichologische Lotion, ein Mittel für weichere Haut, ein Mittel zur Verstärkung der Hauttextur, eine Hautaufhellungsmittelzusammensetzung, eine Reinigungsmittelzusammensetzung, ein seifenhaltiges Mittel, eine Sonnenschutzmittelzusammensetzung, eine Fleckentfernungsmittelzusammensetzung für Textilien, eine Weichspülmittelzusammensetzung, eine Gewebe-Konditionierungs-Zusammensetzung, eine Zusammensetzung gegen verknittertes Gewebe, eine Aromazusammensetzung für Dampfbügeleisen, eine Kerzenstoffzusammensetzung, eine Zusammensetzung zur Regulierung des Pflanzenwachstums, eine Zusammensetzung zur Anregung des Pflanzenwachstums, eine Düngemittelzusammensetzung, eine Insektenanlockmittelzusammensetzung, eine Insektenabstoßmittelzusammensetzung, eine Abflussreinigungsmittelzusammensetzung, eine Schneckenbekämpfungsmittelzusammensetzung, eine Antitranspirationsmittelzusammensetzung, eine Körperdeodorant-Zusammensetzung, eine Körperdeodorant/Antitranspirations-Vorrichtung, ein .Abfallbehälter, eine Vorrichtung zur Lufterfrischung oder eine Lufterfrischungsmittelzusammensetzung ist.

**12.** Verfahren nach Anspruch 11, wobei die übelriechende Quelle ein aliphatisches Halohydrin, aliphatisches Amin, aliphatisches N-Oxid, Dialkylamin, cycloaliphatisches Amin, cycloaliphatisches N-Oxid, cycloolefinisches Amin, cycloolefinisches N-Oxid, cycloaromatisches Amin, cycloaromatisches N-Oxid, Hydroxyalkylamin, Iminverbindung, Amidverbindung, Aminosäure, Polypeptid, modifiziertes antimikrobielles Protein, Diureid, Nitril, aliphatisches Mercaptan, cycloaliphatisches Mercaptan, Mercaptoalkanoik-Säureester, aliphatisches Monosulfid, Disulfid, Trisulfid, Schwefeloxid, Sulfon oder Sulton, cycloaliphatisches Monosulfid, Disulfid, Trisulfid, Schwefeloxid, Sulfon oder Sulton, cycloolefinisches Monosulfid, Disulfid, Trisulfid, Schwefeloxid, Sulfon oder Sulton, cycloaromatisches Monosulfid, Disulfid, Trisulfid, Schwefeloxid, Sulfon oder Sulton, Isothiocyanat, Thiocyanat, Dithiocyanat, Isothiazolon, Isothiazolinon, Thiodiazinethion, Halosulfamat, Arylsulfonamid, nieder-aliphatische Carbonsäure, Phenol, Phosphin, aliphatisches Phosphit oder Phosphonat, cycloaliphatisches Phosphit oder Phosphonat, Arsin, niedriger Alkohol, niedriges Keton, Hopfen, Hopfensäure, Arylpyrazol, Oxazolin, Isocyanurat, Biguanid, Extrakt der Ratanhiawurzel, Hydantoin, Pyrollidon, Pyrollidoncarbonsäure, Pyrollidoncarbonsäureester, Nitrophenol, N-substituierte Asparaginsäure oder Pyrethroid ist.

**13.** Verfahren zum Entgegenwirken eines Gestankes, der aus einer festen oder flüssigen duftstoffhaltigen Seife oder Reinigungsmittel austreten kann, umfassend den Schritt des Einbringens einer Menge und Konzentration der Zusammensetzung nach einem der Ansprüche 1 bis 7, die dem Gestank entgegenwirkt, in die Seife oder in das Reinigungsmittel.

**14.** Einzel-Flüssigphasen-Zusammensetzung, bestehend im Wesentlichen aus 10 bis 30 Gewichtsprozent der Zusammensetzung nach einem der Ansprüche 1 bis 7 und einem Lösungsmittel aus Glycol oder Dipropylenglycol.

**15.** Seife oder Reinigungsmittel, umfassend die Zusammensetzung nach einem der Ansprüche 1 bis 7, wobei die Konzentration der Zusammensetzung von 75 ppm bis 300 ppm beträgt.

## Revendications

1. Composition comprenant :

(i) 10 à 90 parties en poids d'un matériau contenant un composé organique monocyclique substitué, où le matériau contenant le composé organique monocyclique substitué est le propionate de 1-(4'-méthyléthyl)cyclohexyléthan-1-yle ; et
(ii) 90 à 10 parties en poids d'une composition contenant du ricinoléate de zinc, sélectionnée dans le groupe composé du ricinoléate de zinc et d'une solution de ricinoléate de zinc contenant plus de 30 % en poids de ricinoléate de zinc.

2. Composition selon la revendication 1, comprenant en outre un matériau supplémentaire contenant un composé organique monocyclique substitué sélectionné dans le groupe composé du butyrate de 1-cyclohexyléthan-1-yle, de l'acétate de 1-cyclohexyléthan-1-yle et du 1-cyclohexyléthan-1-ol.

3. Composition selon la revendication 1 ou la revendication 2, dans laquelle la composition contenant du ricinoléate de zinc de la partie (ii) est une solution de ricinoléate de zinc contenant environ 50 % en poids de ricinoléate de zinc et environ 50 % en poids d'au moins un 1-hydroxy-2-éthoxyéthyléther d'un alcool en $C_{12}$-$C_{14}$.

4. Composition selon la revendication 1, comprenant un mélange de butyrate de 1-cyclohexyléthan-1-yle et d'acétate de 1-cyclohexyléthan-1-yle.

5. Composition selon la revendication 4, dans laquelle la composition contenant du ricinoléate de zinc définie dans la partie (ii) de la revendication 1 est la solution de ricinoléate de zinc contenant environ 50 % en poids de ricinoléate de zinc et environ 50 % en poids d'au moins un 1-hydroxy-2-éthoxyéthyléther d'un alcool en $C_{12}$-$C_{14}$ et dans laquelle le rapport pondéral entre composition contenant du ricinoléate de zinc, butyrate de 1-cyclohexyléthan-1-yle, acétate de 1-cyclohexyléthan-1-yle et propionate de 1-(4'-méthyléthyl)cyclohexyléthan-1-yle est environ égal à 2:1:1:1.

6. Composition selon la revendication 1, comprenant de l'acétate de 1-cyclohexyléthan-1-yle.

7. Composition selon la revendication 6, dans laquelle la composition contenant du ricinoléate de zinc définie dans la partie (ii) de la revendication 1 est la solution de ricinoléate de zinc contenant environ 50 % en poids de ricinoléate de zinc et environ 50 % en poids d'au moins un 1-hydroxy-2-éthoxyéthyléther d'un alcool en $C_{12}$-$C_{14}$ et dans laquelle le rapport pondéral entre composition contenant du ricinoléate de zinc, acétate de 1-cyclohexyléthan-1-yle et propionate de 1-(4'-méthyléthyl)cyclohexyléthan-1-yle est environ égal à 3:1:1.

8. Procédé pour réduire substantiellement, éliminer ou contrer une mauvaise odeur émanant d'une source malodorante liquide ou solide, comprenant l'étape consistant à fournir une quantité et une concentration qui contrent la mauvaise odeur de la composition selon l'une quelconque des revendications 1 à 7 sous la forme d'une dose unique, d'une dose continue sur une durée contrant la mauvaise odeur, ou d'une dose périodique.

9. Procédé de prévention d'une mauvaise odeur émanant d'une source malodorante solide possédant une surface laminaire définie, comprenant l'étape consistant à appliquer sur ladite surface laminaire définie une quantité et une concentration qui contrent la mauvaise odeur de la composition selon l'une quelconque des revendications 1 à 7.

10. Procédé de prévention d'une mauvaise odeur émanant d'une source malodorante solide ou liquide, comprenant l'étape consistant à ajouter en mélange à ladite source une quantité et une concentration qui contrent la mauvaise odeur de la composition selon l'une quelconque des revendications 1 à 7.

11. Procédé selon l'une quelconque des revendications 8 à 10, dans lequel la source malodorante est un herbicide, une composition antivirale, un fongicide, un bactéricide, un parasiticide, un insecticide, une préparation dépilatoire, une composition de blanchiment, une préparation pour nettoyer les surfaces dures, une composition de nettoyage de la peau, une préparation antimicrobienne pour ongles, un fixant pour cheveux, une composition de conditionnement pour cheveux, une lotion trichologique, une composition pour adoucir la peau, une composition améliorant la texture de la peau, une composition d'éclaircissement du teint, une composition détergente, une composition de savon, une composition d'écran solaire, une composition détachante pour tissu, une composition adoucissant les tissus, une composition de conditionnement du tissu, une composition anti-froissement pour tissu, une composition d'arôme pour le repassage à la vapeur, une composition pour bougie, une composition régulant la croissance

végétale, une composition stimulant la croissance végétale, une composition fertilisante, une composition attirant les insectes, une composition répulsive pour insectes, une composition de nettoyage des canalisations, une composition molluscicide, une composition antiperspirante, une composition de déodorant corporel, un dispositif de déodorant corporel / antiperspirant, un conteneur d'évacuation des déchets, un dispositif pour rafraîchir l'air ou une composition pour rafraîchir l'air.

**12.** Procédé selon la revendication 11, dans lequel la source malodorante est une halohydrine aliphatique, une amine aliphatique, un N-oxyde aliphatique, une dialkylamine, une amine cycloaliphatique, un N-oxyde cycloaliphatique, une amine cyclooléfinique, un N-oxyde cyclooléfinique, une amine cycloaromatique, un N-oxyde cycloaromatique, une hydroxyalkylamine, un composé imine, un composé amide, un acide aminé, un polypeptide, une protéine antimicrobienne modifiée, un diuréide, un nitrile, un mercaptan aliphatique, un mercaptan cycloaliphatique, un acide mercaptoalcanoïque, un ester d'acide mercaptoalcanoïque, un monosulfure, disulfure, trisulfure, oxyde de soufre, sulfone ou sultone aliphatique, un monosulfure, disulfure, trisulfure, oxyde de soufre, sulfone ou sultone cycloaliphatique, un monosulfure, disulfure, trisulfure, oxyde de soufre, sulfone ou sultone cyclo-oléfinique, un monosulfure, disulfure, trisulfure, oxyde de soufre, sulfone ou sultone cycloaromatique, un isothiocyanate, un thiocyanate, un dithiocyanate, une isothiazolone, une isothiazolinone, une thiodiazinethione, un halosulfamate, un arylsulfonamide, un acide carboxylique aliphatique inférieur, un phénol, une phosphine, un phosphite ou un phosphonate aliphatique, un phosphite ou un phosphonate cycloaliphatique, une arsine, un alcool inférieur, une cétone inférieure, des houblons, de l'acide de houblon, un arylpyrazole, une oxazoline, un isocyanurate, un biguanide, un extrait de krameria, une hydantoïne, une pyrrolidone, un acide carboxylique de pyrrolidone, un ester d'acide carboxylique de pyrrolidone, un nitrophénol, un acide aspartique N-substitué ou un pyréthroïde.

**13.** Procédé pour contrer une mauvaise odeur émanant d'un savon ou détergent solide ou liquide contenant du parfum, comprenant l'étape consistant à introduire dans le savon ou le détergent une quantité et concentration qui contrent la mauvaise odeur de la composition selon l'une quelconque des revendications 1 à 7.

**14.** Composition à phase liquide unique contenant essentiellement 10 à 30 % en poids de la composition selon l'une quelconque des revendications 1 à 7 et un solvant de glycol ou de dipropylèneglycol.

**15.** Savon ou détergent comprenant la composition selon l'une quelconque des revendications 1 à 7, dans lequel la concentration de la composition est comprise entre 75 ppm et 300 ppm.

FIG. 1

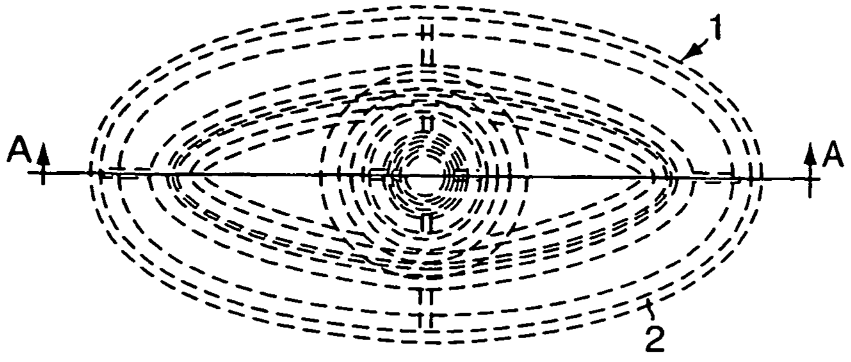


FIG. 2

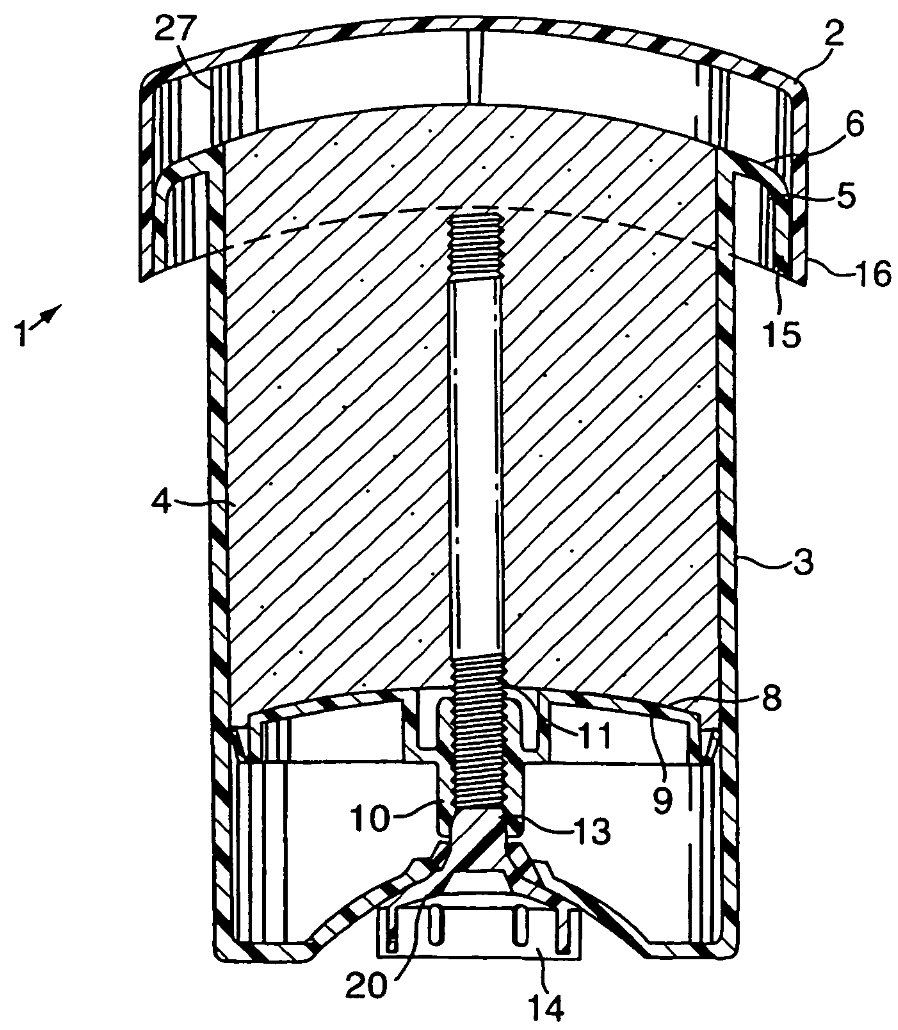

FIG. 3

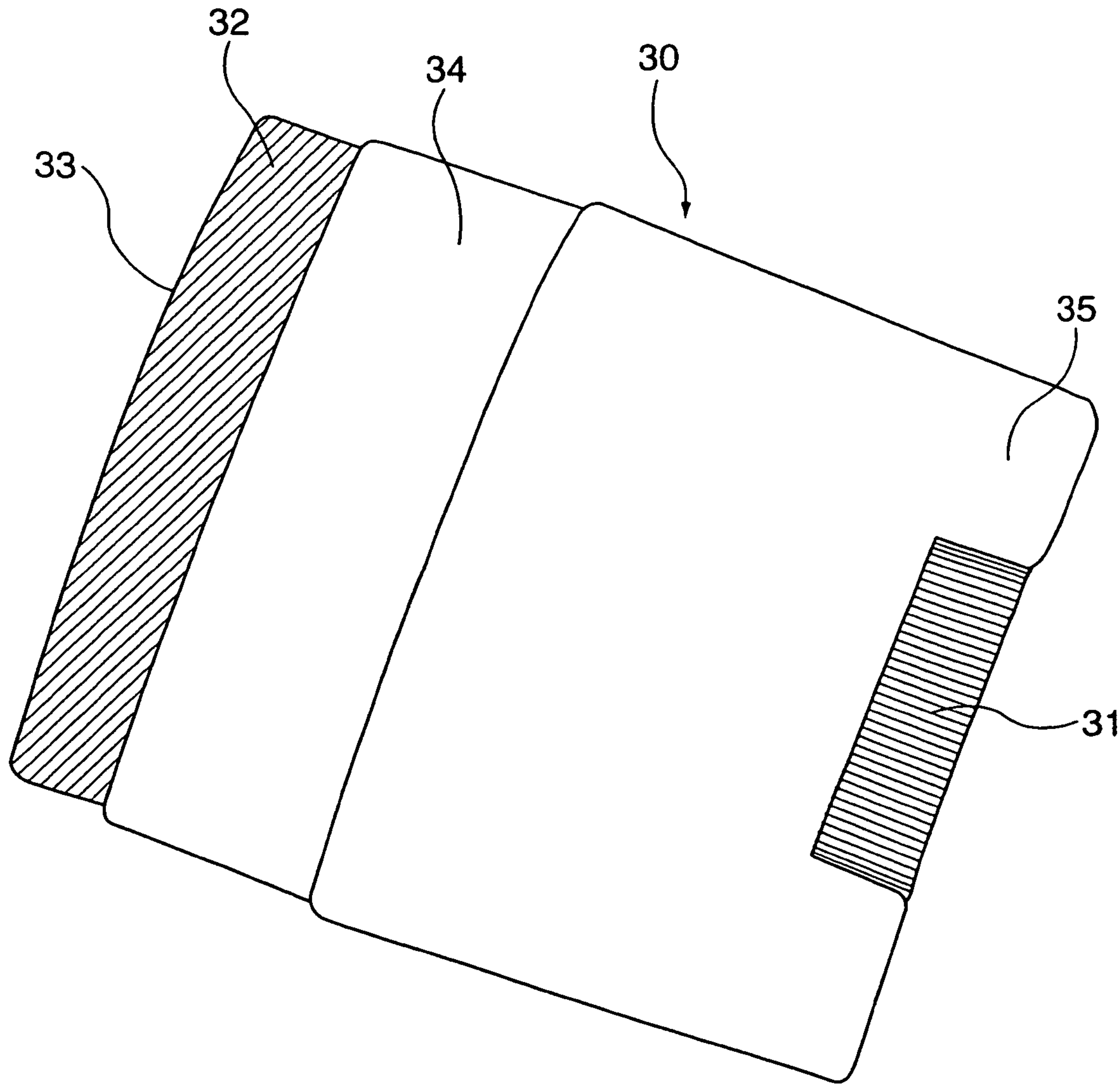

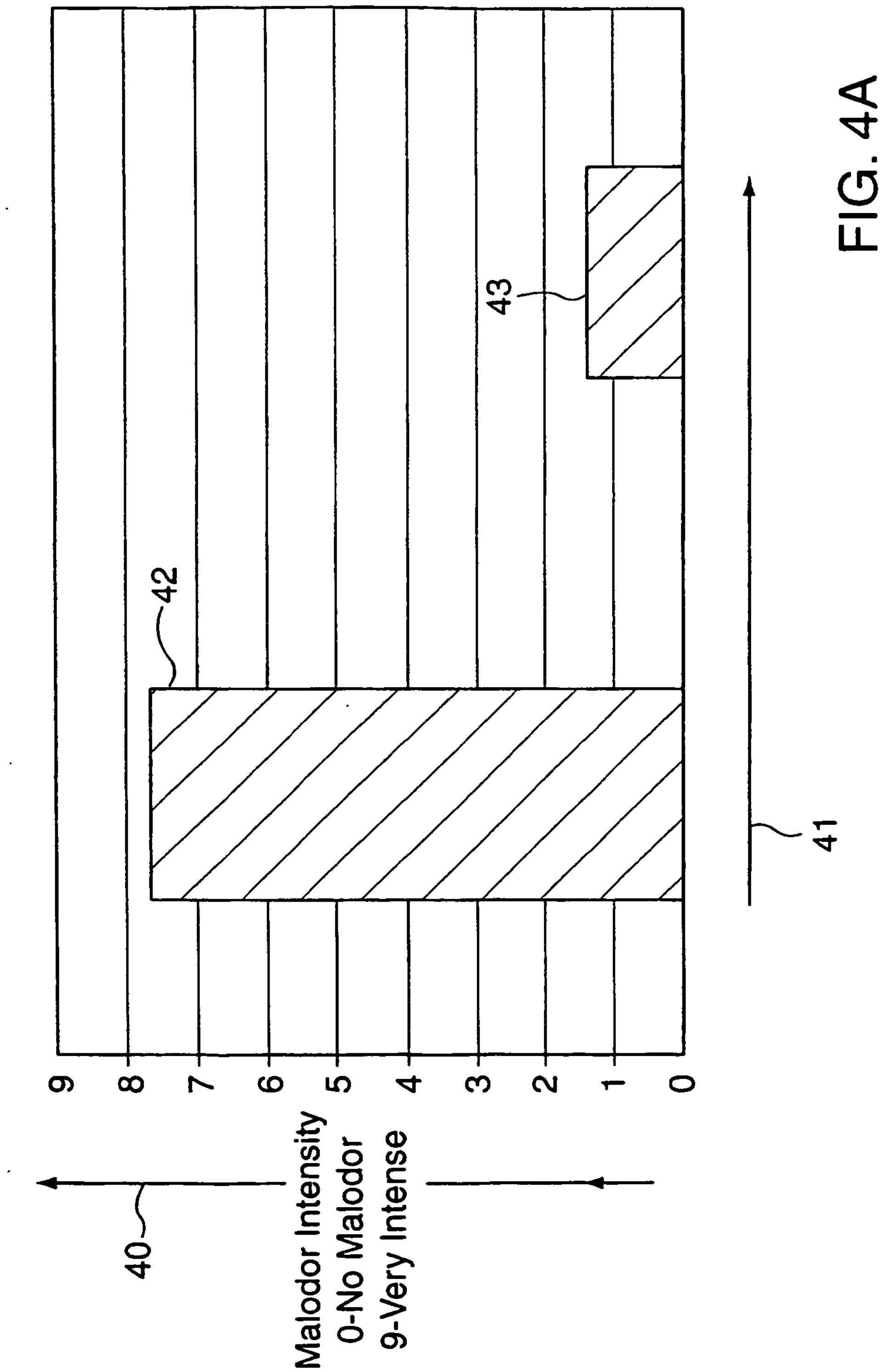
Malodor Intensity
0-No Malodor
9-Very Intense
0
1
2
3
4
5
6
7
8
9
40
41
42
43

FIG. 4A

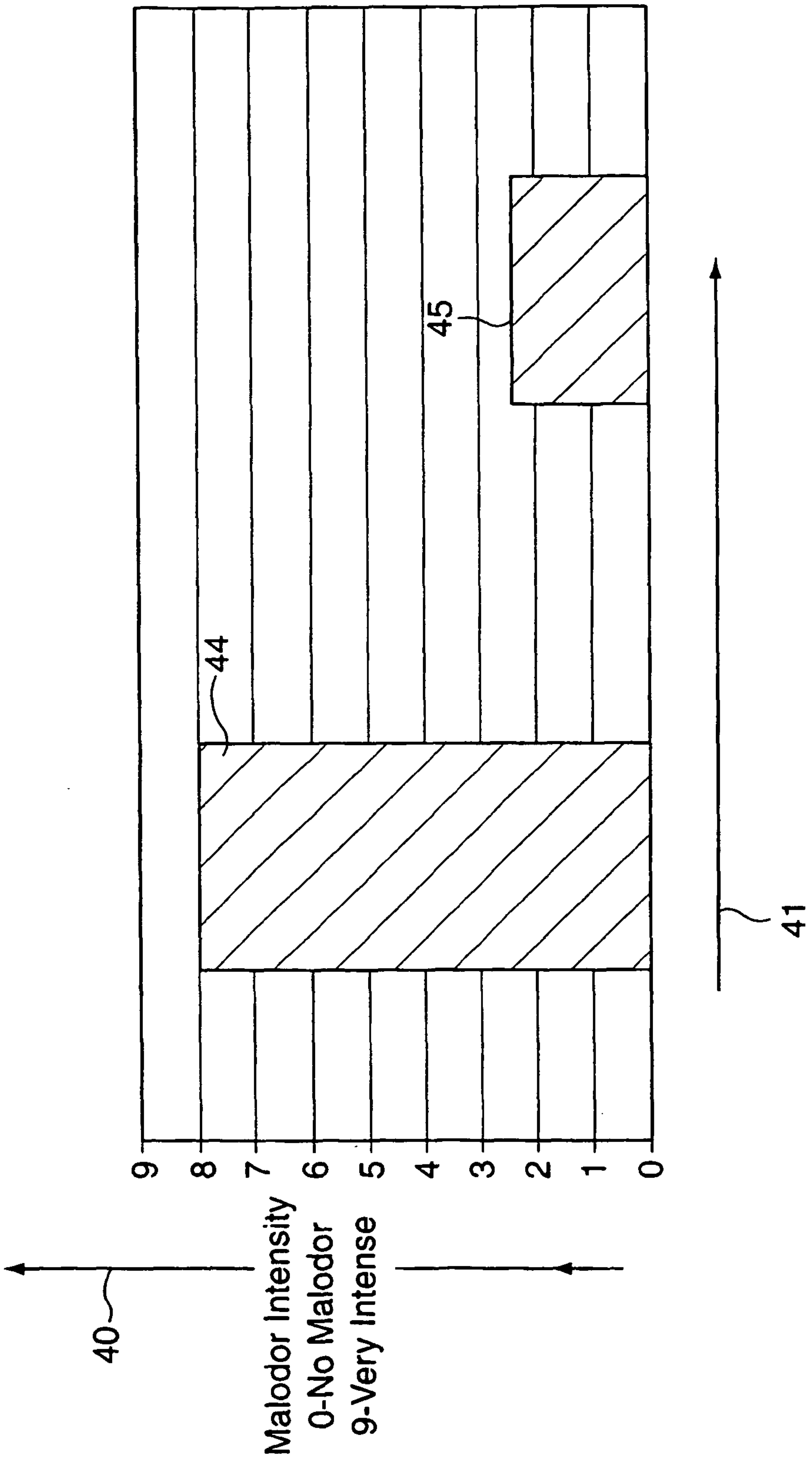
Malodor Intensity
0-No Malodor
9-Very Intense
0
1
2
3
4
5
6
7
8
9
40
41
44
45

FIG. 4B

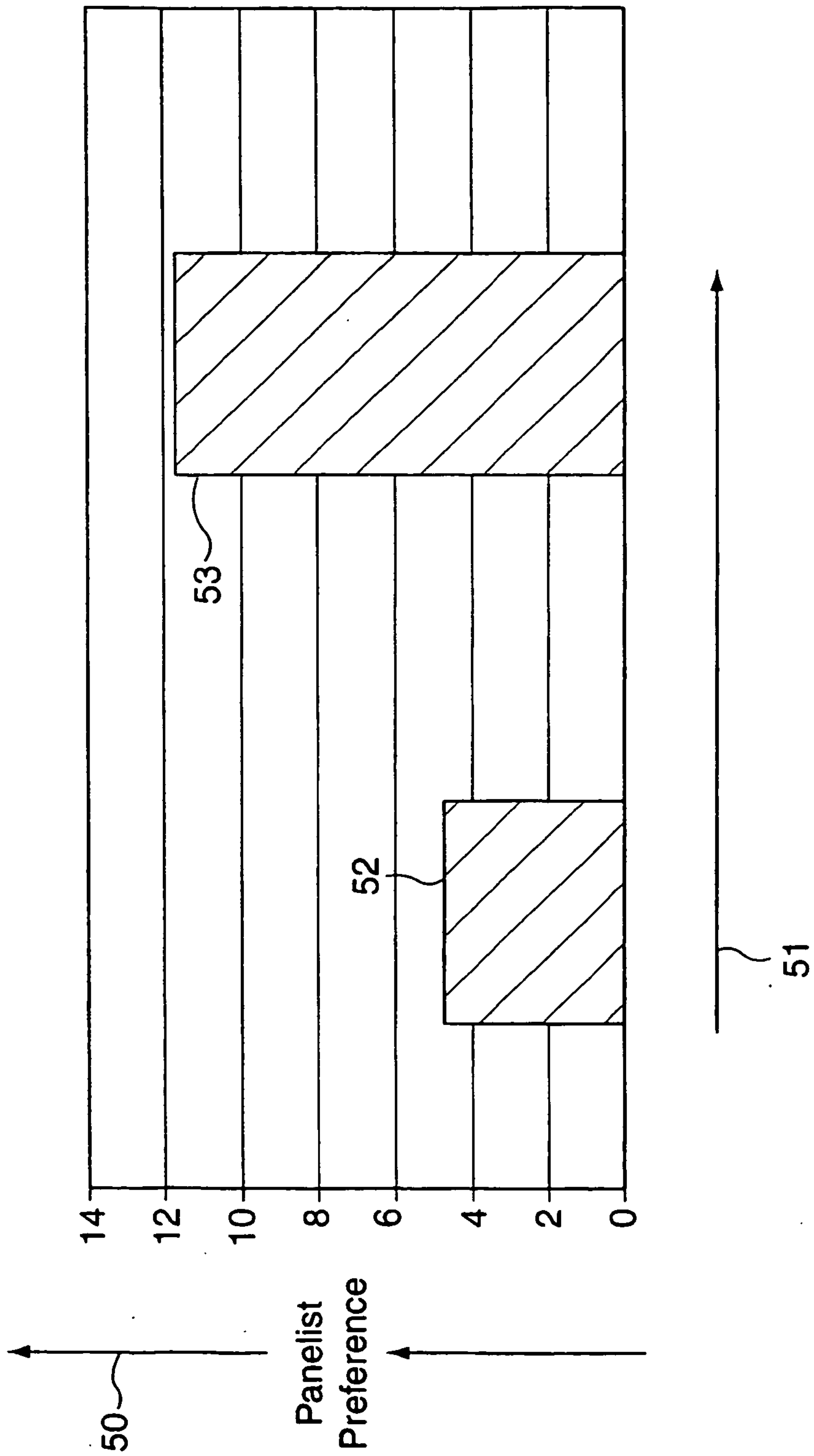


FIG. 5

FIG. 6

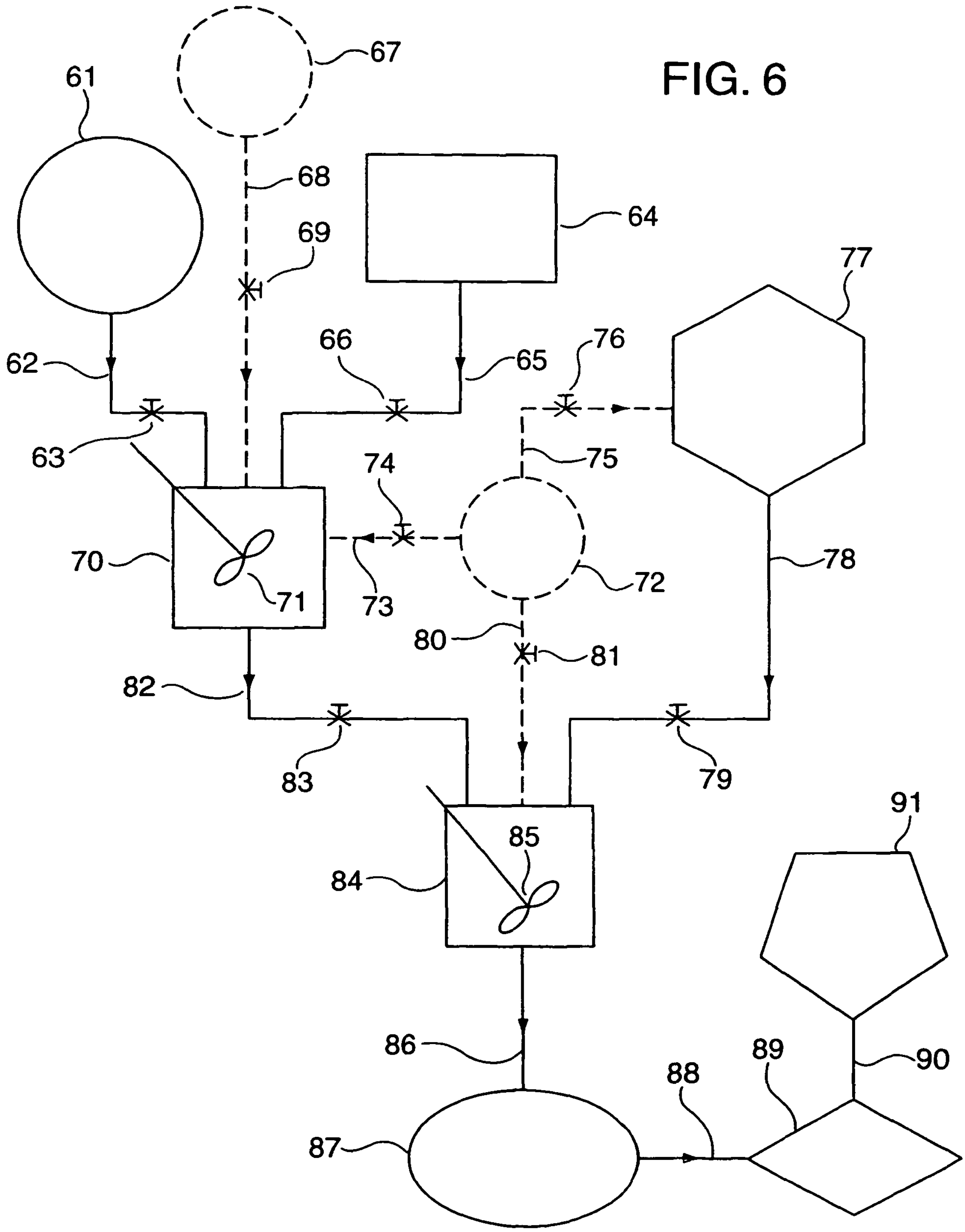
61
62
63
64
65
66
67
68
69
70
71
72
73
74
75
76
77
78
79
80
81
82
83
84
85
86
87
88
89
90
91

FIG. 7

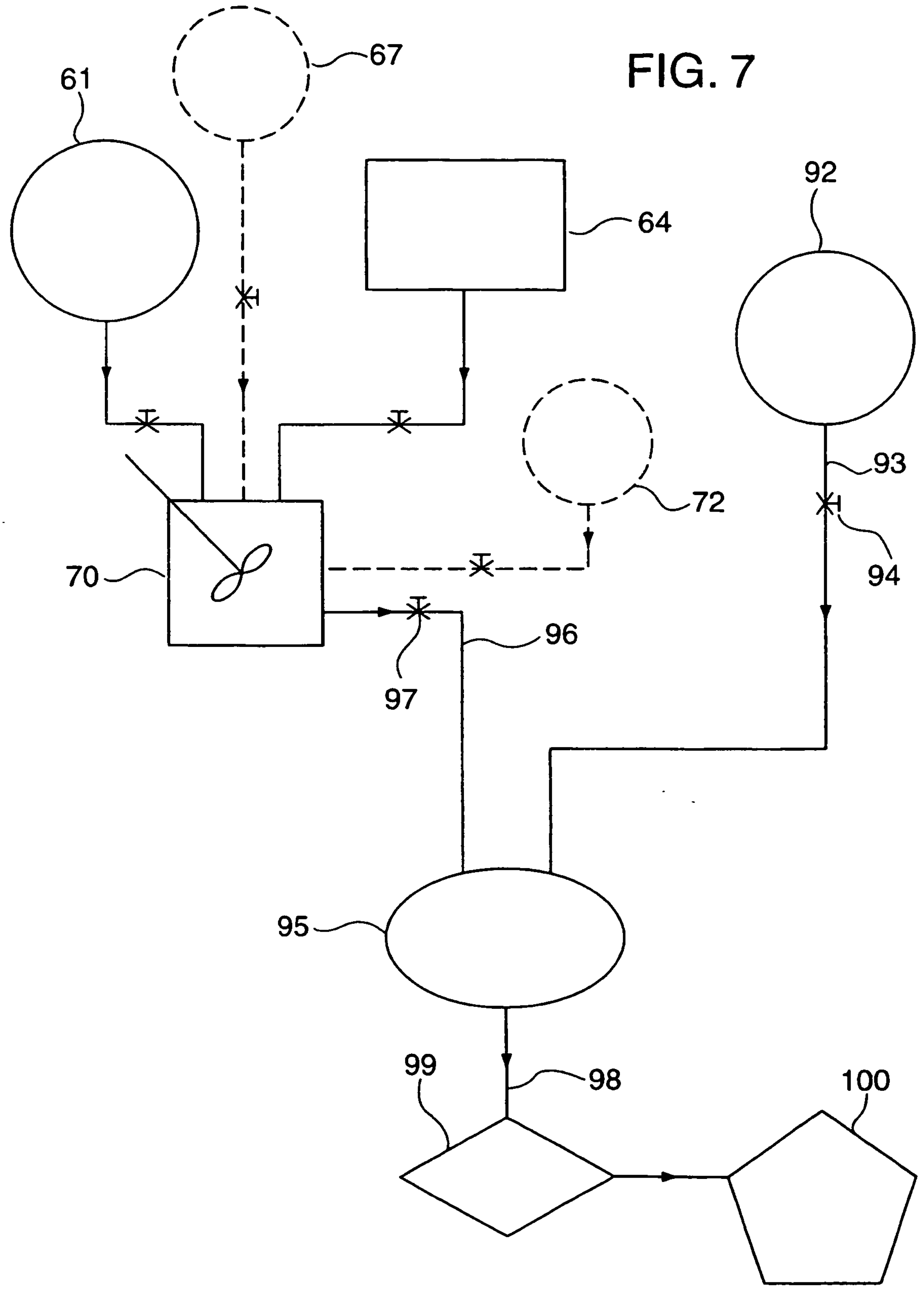

FIG. 8

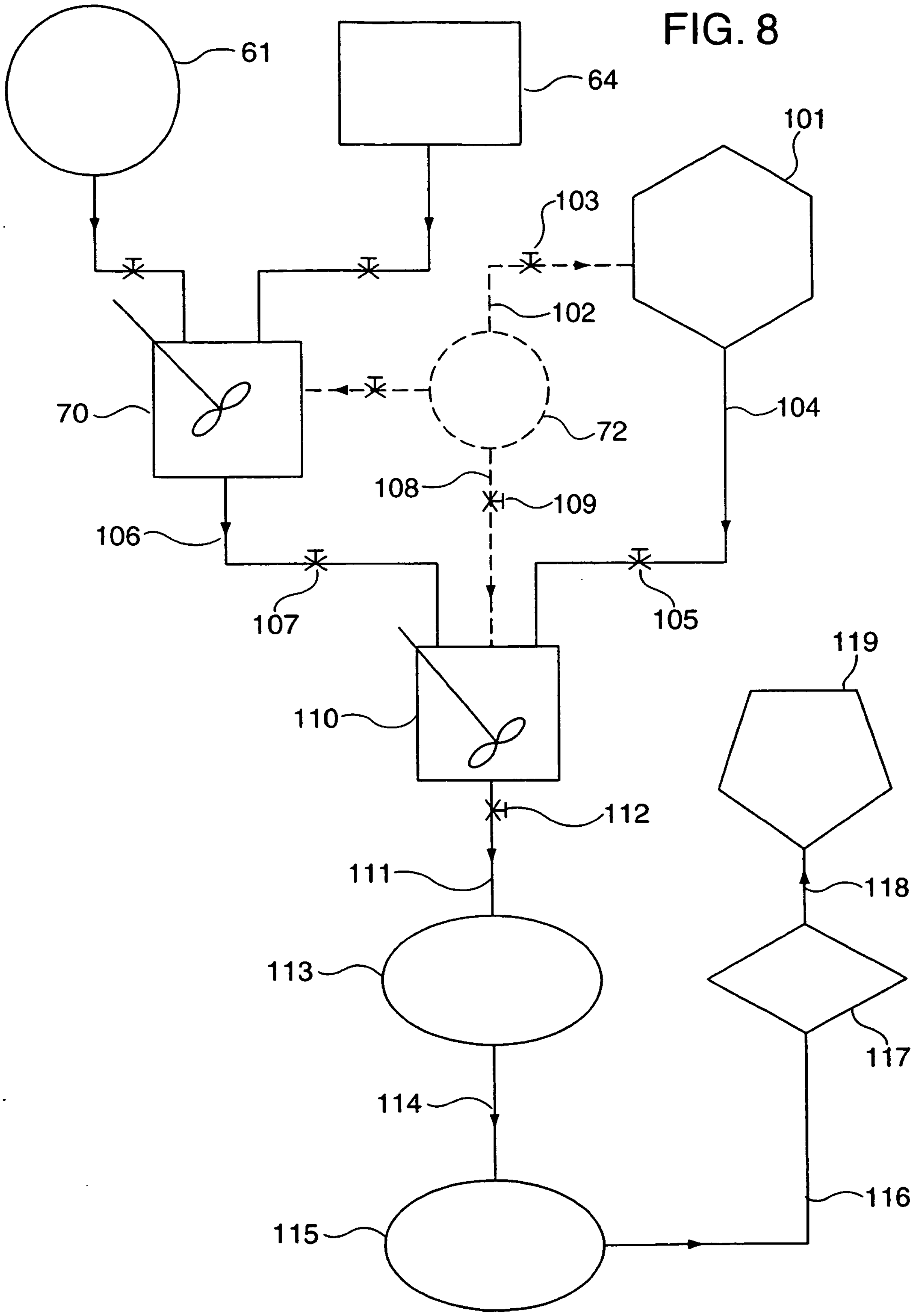
61
64
101
103
102
70
72
104
108
109
106
107
105
119
110
112
111
118
113
117
114
116
115

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6528047 B
- US 6592813 B
- US 4968496 A
- US 2003007945 A1
- US 6432891 B
- US 4622221 A
- US 683525 A
- US 6495512 B
- US 6517759 B
- US 20030110682 A1
- US 5348667 A
- US 5652206 A
- US 6528013 B
- US 5783657 A
- US 6552160 B
- US 6268466 B
- US 6469131 B
- US 20030114323 A1